# EUROPEAN PATENT APPLICATION

(11) **EP 4 428 233 A1**
(43) Date of publication of application: **11.09.2024**
(21) Application number: 22900290.2
(22) Date of filing: 17.11.2022
(51) Int. Cl.: C12N 9/22, C12N 15/10

(54) **C2C9 NUCLEASE-BASED NOVEL GENOME EDITING SYSTEM AND APPLICATION THEREOF**

(30) Priority: 30.11.2021 CN 202111445474
(71) Applicant: Shanghaitech University, Shanghai 201210 (CN)
(72) Inventor: JI, Quanjiang, Shanghai 201210 (CN); CHEN, Weizhong, Shanghai 201210 (CN); MA, Jiacheng, Shanghai 201210 (CN)
(74) Representative: Canzler & Bergmeier Patentanwälte Partnerschaft mbB
(86) International application number: PCT/CN2022/132596
(87) International publication number: WO 2023/098485

(57) **Abstract**

The present invention relates to the field of biomedicine, and disclosed are a use of a C2C9 nuclease as an RNA-guided endonuclease, and an extremely small genome editing system. The genome editing system comprises a C2C9 nuclease and/or a nucleic acid encoding the C2C9 nuclease, and a guide RNA or a nucleic acid encoding the guide RNA. The extremely small genome editing system of the present invention can perform gene editing on at least one target sequence in a genome of prokaryotic bacteria and eukaryotic cells. Also disclosed in the present invention are an application of the genome editing system and an editing method. By means of the genome editing system or method of the present invention, a target gene can be precisely knocked out or cut off, the cutting efficiency is high, and precise editing of the target gene is implemented.

## Description

### FIELD OF DISCLOSURE

The present disclosure belongs to the field of biomedicine and relates to a novel genome editing system based on an extremely small C2C9 nuclease, as well as to methods and applications of the system for genome editing in prokaryotic bacteria and eukaryotic cells.

### BACKGROUND OF RELATED ARTS

Clustered regularly interspaced short palindromic repeats/CRISPR-associated protein (CRISPR/Cas) is a novel genome editing system developed in recent years. The system mainly includes a Cas endonuclease and the corresponding guide RNA. Cas endonuclease specifically binds to the guide RNA and then uses base complementary pairing to target, recognize, and cleave specific sites in the genome, causing double-stranded breaks of genomic DNA. The broken genomic DNA is then repaired using the organism's endogenous or exogenous DNA repair mechanisms, such as homologous recombination and non-homologous recombination end-joining, thereby enabling the editing of specific sites in the genome during the repair process.

Genetic manipulation of cells or species using the CRISPR/Cas system is widely applied in basic research, biotechnology development, and disease therapeutics development across the fields of life sciences, medicine, and agronomy. For example, it can be used to correct mutated genes responsible for genetic or cancer, perform genetic engineering of crops to enhance their performance, precisely modify microbial genomes to promote the production of high value-added compounds, cleave the genomes of pathogenic microorganisms to kill them for infection treatment, etc. Due to its simplicity and efficiency, the CRISPR/Cas system has been widely used in the fields of medicine, biology, and agronomy.

The two main types of CRISPR/Cas genome editing systems widely used today include CRISPR/Cas9 and CRISPR/Cas12a. In these two types of genome editing systems, the CRISPR effector protein nucleases Cas9 and Cas12a are large proteins containing more than 1,000 amino acids, which leads to significant challenges for the delivery of CRISPR/Cas9 or CRISPR/Cas12a into cells. For example, CRISPR/Cas9 or CRISPR/Cas12a systems often need to be packaged into adeno-associarted virus (AAV) vectors for use during in vivo gene therapy. However, AAV vectors have limited packaging capacity. The large molecular size of CRISPR/Cas9 and CRISPR/Cas12a systems greatly reduces the AAV vector packaging efficiency, increases the difficulty of packaging, and greatly restricts the wide application of these systems in gene therapy and other fields. In addition, the protospacer adjacent motif (PAM, PAM sequences, or PAM sites) recognized by the CRISPR/Cas system restricts the genomic sites that can be selected, hampering its in-depth application in fields such as medicine and biology. Therefore, the development of new, efficient, and small sized genome editing systems is particularly urgent.

### SUMMARY OF THE PRESENT DISCLOSURE

The present disclosure provides a small sized genome editing system (including a CRISPR/C2C9 system) and a method. The genome editing system of the present disclosure can be utilized to accurately cleave target sites in prokaryotic bacteria and eukaryotic cells, thereby realizing precise knockout or editing of genes. The present disclosure also provides a novel RNA-programmable endonuclease. The present disclosure also relates to other components, including guide RNA (gRNA) and/or target sequence, a method for generating and using them in various applications. Examples of such applications include a method for regulating transcription, as well as a method for targeting, editing, and/or manipulating genes (e.g., DNA or RNA) using a novel nuclease and other components (such as nucleic acids and/or polypeptides). The present disclosure also relates to a recombinant cell, a kit, and the like comprising elements of the editing system.

In one aspect, the present disclosure provides a genome editing system for editing at least one target sequence in a genome, including:
(a) a C2C9 nuclease or a nucleic acid encoding the C2C9 nuclease; and
(b) a guide RNA (gRNA) or a nucleic acid encoding the gRNA.

The C2C9 nuclease and the matching guide RNA form a complex.

In a preferred embodiment, the genome editing system is a CRISPR-C2C9 genome editing system.

In a preferred embodiment, the CRISPR-C2C9 genome editing system includes:
(1) an expression construct of the C2C9 nuclease;
(2) an expression construct of a complete guide RNA.

The complete guide RNA comprises a guide RNA backbone, and a RNA sequence corresponding to a target sequence at the 3' end of the guide RNA backbone. The target sequence is a nucleic acid fragment that is 12 to 40 base pairs (bp) in length following the PAM sequence, preferably a nucleic acid fragment that is 20 bp in length following the PAM sequence. The C2C9 nuclease described in the present disclosure may be a conventional C2C9 nuclease. Preferably, the C2C9 nuclease contains no greater than 800 amino acids. More preferably, the C2C9 nuclease has an amino acid sequence that is any of SEQ ID NOs. 3-117 or an amino acid sequence that is at least 80% identical to any of SEQ ID NOs. 3-117. In some embodiments, the C2C9 nuclease is selected from the group consisting of *Actinomadura craniellaeC2C9* (AcC2C9), *Corynebacterium glutamicumC2C9* (CgC2C9), *Rothia dentocariosaC2C9* (RdC2C9), *Micrococcus luteusC2C9* (MilC2C9), etc.

In a preferred embodiment of the present disclosure, the C2C9 nuclease has an amino acid sequence shown as SEQ ID NO. 3.

In some embodiments, the nucleic acid encoding the AcC2C9 nuclease is deoxyribonucleic acid (DNA). In some embodiments, a DNA encoding sequence for AcC2C9 nuclease is shown as SEQ ID NO. 121 or a variant having at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID NO. 121.

In some embodiments, the nucleic acid encoding the AcC2C9 nuclease is ribonucleic acid (RNA). In some embodiments, the RNA encoding the C2C9 nuclease is mRNA.

In some embodiments, the sequence encoding the AcC2C9 nuclease is an optimized coding sequence for human codons, preferably shown as SEQ ID NO. 122 or a variant having at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, or at least 99% sequence identity to SEQ ID NO. 122.

In some embodiments, the C2C9 nuclease or nucleic acid encoding C2C9 nuclease is formulated in liposomes or lipid nanoparticles. In some embodiments, the liposome or lipid nanoparticle further comprises the gRNA or the nucleic acid encoding the gRNA.

In some embodiments, the system comprises a C2C9 nuclease pre-complexed with gRNA, forming a ribonucleoprotein (RNP) complex that recognizes a PAM sequence on a target gene (e.g., target DNA) sequence. That is, the precise localization of the DNA sequence flank includes recognizing the PAM sequences on the target gene sequence by the complex containing C2C9 nuclease and guide RNA.

The guide RNA backbone of the AcC2C9 nuclease has a sequence shown as SEQ ID NO. 120.

In some embodiments, the complete guide RNA expression construct comprises a first DNA sequence for expressing the guide RNA backbone, and a second DNA sequence for expressing a RNA sequence corresponding to a target sequence, where the second DNA sequence is added at the 3' end of the first DNA sequence, and the target sequence is a fragment of 20 bp in length following the PAM sequence.

The PAM site preferably recognized by the CRISPR-C2C9 genome editing system is AAN and/or GAN.

In certain embodiments, the system described herein may further comprise a donor template including a heterologous polynucleotide sequence, wherein the heterologous polynucleotide sequence is capable of being inserted into a target polynucleotide sequence. In some embodiments, the heterologous polynucleotide donor template is physically linked to the gRNA.

In another aspect, the present disclosure provides a method for targeting, editing, modifying, or manipulating double-stranded DNA at a target motif, where the method comprises mixing the C2C9 nuclease and the complete guide RNA as described above with the double-stranded DNA. The complete guide RNA is: 5'- the guide RNA backbone corresponding to the C2C9 nuclease + a RNA sequence corresponding to a target sequence on the double-stranded DNA - 3'. In some embodiments, the method is used to cleave the double-stranded DNA.

In some embodiments, the present disclosure provides a method for targeting, editing, modifying, or manipulating double-stranded DNA at a target motif in a cell. In some embodiments, the method further comprises a step of introducing a polynucleotide donor template into the cell, where the donor template may be a donor single-stranded or double-stranded polynucleotide donor template.

In some embodiments of the present disclosure, DNA at DSBs is repaired by homology-directed repair, non-homologous end joining, or microhomology-mediated end joining.

As described above, the guide RNA backbone corresponding to the AcC2C9 nuclease preferably has a sequence shown as SEQ ID NO. 120. Complete guide RNAs can be formed by adding target RNA sequences against different target sequences at the 3' end of the guide RNA backbone.

The C2C9 nuclease is preferably selected from the group consisting of SEQ ID NOs. 3-117. More preferably, the C2C9 nuclease is selected from *Actinomadura craniellae* C2C9 (AcC2C9), *Corynebacterium glutamicum* C2C9 (CgC2C9), *Rothia dentocariosa* C2C9 (RdC2C9), and *Micrococcus luteus* C2C9 (MiC2C9). More preferably, the C2C9 nuclease has a sequence shown as SEQ ID NO. 3 or is a variant thereof. The encoding sequence of the AcC2C9 nuclease optimized for better targeting of human codons is shown as SEQ ID NO. 122. The variant has at least 20% (e.g., 30%, 40%, 50%, 60%, 70%, 80%, 85%, 90%, 91%, 92%, 93%, 94%, 95%, 96%, 97%, 98%, or 99%) sequence identity to the amino acid residues of SEQ ID NO. 3 and retains RNA-guided DNA binding activity and/or double-stranded DNA cleavage activity.

The cleavage method described in the present disclosure may be conventional in the art, e.g., in a preferred embodiment of the present disclosure, the cleavage is carried out in the presence of 50 to 200 mM NaCl and/or 5 to 20 mM MgCl₂. For example, the concentration of NaCl is 50 to 100 mM, 100 to 150 mM, or 150 to 200 mM. For example, the concentration of MgCl₂ is 5 to 10 mM, 10 to 15 mM, or 15 to 20 mM. Preferably, the cleavage is carried out in a buffer solution containing 150 mM NaCl, 10 mM MgCl₂, 10 mM Tris-HCl, pH=7.5, and 1 mM DTT.

The temperature of the cleavage is 34 to 43°C, for example, 34 to 37°C, 37 to 40°C, or 40 to 43°C, preferably 37°C. The time of the cleavage is preferably 30-60 min.

In another aspect, the present disclosure provides a method for genome editing, comprising introducing the genome editing system as described above into a prokaryotic bacterial or eukaryotic cell containing a target sequence to perform genome editing.

The prokaryotic bacteria described in the present disclosure include, but are not limited to, prokaryotic microorganisms, such as Escherichia coli, Klebsiella pneumoniae, and the like.

The eukaryotic cells described in the present disclosure include, but are not limited to, mammalian cells, yeast, and other eukaryotic cells.

The "genome editing" in the above method for genome editing may be conventional in the art, such as, but not limited to, gene cleavage, gene deletion, gene insertion, point mutation, transcription inhibition, transcription activation, base editing, and prime editing.

In another aspect, the present disclosure provides a use of the C2C9 nuclease in genome editing.

The C2C9 nuclease and the genome editing are as described above.

The nuclease in the genome editing system comprises no greater than 800 amino acids.

In another aspect, the present disclosure provides a modified cell, obtained by editing the genome of a cell using any of the systems or methods described above. In one embodiment, the modified cell includes: (a) the C2C9 nuclease or the nucleic acid encoding the C2C9 nuclease described above, and (b) the guide RNA (gRNA) or the nucleic acid encoding the gRNA, wherein the gRNA is capable of directing the C2C9 nuclease or a variant thereof to a target polynucleotide sequence. In certain embodiments, the modified cell further comprises a donor template including a heterologous polynucleotide sequence, wherein the heterologous polynucleotide sequence is capable of being inserted into the target polynucleotide sequence.

The present disclosure has the following beneficial effects:

CRISPR/C2C9 is a new genome editing system developed by the applicant, including a C2C9 nuclease and a gRNA. The system features a small size (the C2C9 nuclease generally includes no more or even less than 800 amino acids) and does not require complex PAM (the PAM site to be recognized is AAN, etc.). Through the guidance and localization functions of complete guide RNA, the C2C9 nuclease is able to precisely locate, target, and cleave genomic DNA in vivo, in isolated cells, or in a cell-free environment, so as to achieve genomic DNA double-strand breaks. Utilizing the host cell's own or exogenously supplemented repair mechanisms, the system enables highly efficient and precise genome editing in vivo, in isolated cells, or in cell-free environments.

### BRIEF DESCRIPTION OF THE DRAWINGS

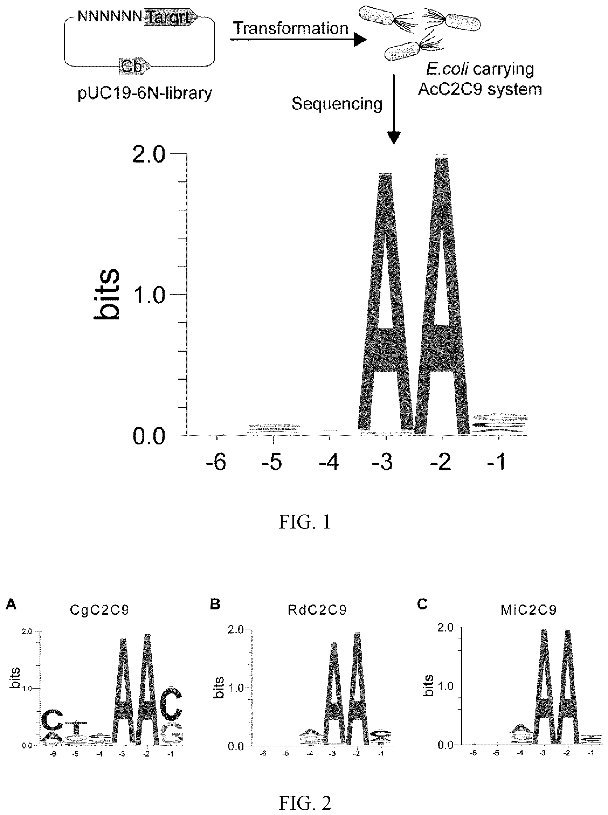

Figure 1 shows an identification process and identification results of PAM of AcC2C9. The results show that AcC2C9 can effectively recognize 5'-AAN type PAM sequences as well as a small number of 5'-GAN sequences (where N is a degenerate base, representing any one of the bases of A, T, C, and G). The horizontal coordinates -1, -2, -3, -4, -5, and -6 respectively represent the 1st, 2nd, 3rd, 4th, 5th, and 6th DNA bases located upstream of the 5' end of a target-DNA segment sequence on an NTS strand (non-targeting strand). The letters on these coordinates represent the preference of the AcC2C9 nuclease for the DNA sequence, the larger the proportion of the letter, the stronger the preference of AcC2C9 for that base.
Figure 2 shows PAM identification results of CgC2C9, RdC2C9, and MiC2C9. The results show that CgC2C9 (A) can effectively recognize 5'-AAS type PAM sequences (where S is a degenerate base, representing any one of the bases of C and G), and RdC2C9 (B) and MiC2C9 (C) can effectively recognize 5'-AAH type PAM sequences (where H is a degenerate base, representing any one of the bases of A, T, and C).
Figure 3 shows PCR results of gene knockout in live Klebsiella pneumoniae cells using CRISPR-AcC2C9 system. The results show that the selected dha gene is precisely knocked out by the CRISPR-AcC2C9 system.
Figure 4 shows cleavage results of a substrate DNA using AcC2C9 nuclease. The results show that the substrate DNA is precisely cleaved into two DNA fragments by AcC2C9.
Figure 5 shows reaction activities of AcC2C9 nuclease at different temperatures (A), different NaCl concentrations (B), different MgCl₂ concentrations (C), and different divalent ions (D). The results show that AcC2C9 has a highest activity at 40°C, 100 mM NaCl, and 10 mM MgCl₂.
Figure 6 (A) is a schematic sequence of a complete guide RNA for AcC2C9. Figure (B) shows cleavage results of substrate DNA using AcC2C9 nuclease under tracrRNA and crRNA conditions.
Figure 7 shows cleavage results of four fluorescently labeled DNA substrates with 58 bp in length using AcC2C9 nuclease. The results show that the major cleavage site of AcC2C9 on the targeting strand is 21-22 nt downstream of the 3' end of the PAM site, whereas the major cleavage site of AcC2C9 on the non-targeting strand is 15-16 nt downstream of the 3' end of the PAM site.
Figure 8 shows gene editing results in human cells mediated by transient expression plasmids of AcC2C9. Figure 8(A) shows TBE-PAGE gel plot of the Indel experiment. Among the five selected different gene sites, AcC2C9 nuclease successfully achieved efficient genome editing for the VEGFA, HEXA, and DNMT1. Figure 8(B) shows statistical results of high-throughput sequencing after VEGFA gene editing in human cells mediated by transient expression plasmids of AcC2C9. It can be seen that AcC2C9 nuclease can precisely introduce insertions or deletions to the target sequence.
Figure 9 shows gene editing efficiency at 47 loci in HEK293T cells using transient expression plasmids of AcC2C9.
Figure 10 shows editing efficiency at three different gene sites in human cells (HEK293T, HLEA, U-2 OS, Huh-7, and HepG) using AAV expressing the AcC2C9 system.

### DETAILED DESCRIPTION OF THE PREFERRED EMBODIMENTS

The present disclosure will be further described below with reference to specific embodiments. It should be understood that the embodiments are merely for describing the present disclosure instead of limiting the scope of the present disclosure. It should also be understood that after reading what is taught herein, one skilled in the art may make various alterations or modifications to the present disclosure, and these equivalent forms likewise fall within the scope limited by the claims appended to this application.

The terms "C2C9", "C2C9 nuclease", "C2C9 polypeptide", and "C2C9 protein" are used interchangeably herein.

The terms "guide RNA", "gRNA", "single gRNA", and "chimeric gRNA" are used interchangeably herein.

It is to be noted that the term "a" or "an" entity refers to one or more of that entity. Additionally, the terms "a" (or "an"), "one or more," and "at least one" can be used interchangeably herein.

The terms "homology", "identity", or "similarity" refer to sequence similarity between two peptides or two nucleic acid molecules. Homology can be determined by comparing corresponding positions in different polypeptides or nucleic acid molecules. When the same position in different sequences of the compared molecules is occupied by the same base or amino acid, then the molecule is homologous at that position. The degree of homology between sequences is determined by the number of matching or homologous positions shared by the sequences. "Unrelated" or "non-homologous" sequences should have less than 20% homology with one of the sequences disclosed in the present disclosure.

A polynucleotide or polynucleotide region (or polypeptide or polypeptide region) having a certain percentage (e.g., 20%, 30%, 40%, 50%, 60%, 70%, 80%, 90%, 95%, 98%, or 99%) of sequence identity to another polynucleotide or polynucleotide region (or polypeptide or polypeptide region) means that, the two aligned sequences have that percentage of bases (or amino acids) that are identical. This alignment and percentage homology or sequence identity can be determined using software programs and methods known in the art. For example, as described in Ausubel et al. eds. (2007) Current Protocols in Molecular Biology. Preferably, default parameters should be used for sequence alignment. One alternative alignment program is BLAST, which uses default parameters. In particular, when the programs BLASTN and BLASTP are used for alignment, the following default parameters are used: Genetic code = standard; filter = none; strand = both; cutoff = 60; expect = 10; Matrix = BLOSUM62; Descriptions = 50sequences; sort by = HIGH SCORE; Databases = non-redundant, GenBank + EMBL + DDBJ + PDB + GenBank CDS translations + SwissProtein + SPupdate + PIR. Polynucleotides that are considered equivalent in a biological sense are those polynucleotides that have the aforementioned percentage homology and encode polypeptides having the same or similar biological activity.

When the polynucleotide is DNA, a polynucleotide sequence consists of letters representing the following four nucleotide bases: adenine (A), cytosine (C), guanine (G), and thymine (T). When the polynucleotide is RNA, a polynucleotide sequence consists of letters representing the following four nucleotide bases: adenine (A), cytosine (C), guanine (G), and uracil (U). Thus, the term "polynucleotide sequence" is represented by letters corresponding to different nucleotide bases. These letters can be entered into a database of a computer having a central processing unit and used for bioinformatics applications such as functional genomics and homology searches. The term "polymorphism" refers to the coexistence of more than one form of a gene or a part thereof, and the term "polymorphic region of a gene" refers to a region where the gene has different nucleotide manifestations at the same position (i.e., different nucleotide sequences). Gene polymorphic regions can be single nucleotides which are different in various alleles.

In the present disclosure, the terms "polynucleotide" and "oligonucleotide" are used interchangeably, and they refer to polymerized forms of nucleotides in any length, whether deoxyribonucleotides, ribonucleotides, or analogues thereof. Polynucleotides can have any three-dimensional structures and can perform any known or unknown functions. Examples of polynucleotides include, but are not limited to, genes or gene fragments (including probes, primers, EST or SAGE tags), exons, introns, messenger RNAs (mRNAs), transfer RNAs, ribosomal RNAs, ribozymes, cDNAs, dsRNAs, siRNAs, miRNAs, recombinant polynucleotides, branched polynucleotides, plasmids, vectors, isolated DNA of any sequence, isolated RNA of any sequence, and nucleic acid probes and primers. Polynucleotides also comprise modified nucleotides, such as methylated nucleotides and nucleotide analogs. If the polynucleotide is modified, the modification may be conferred before or after the assembly of the polynucleotide. Nucleotide sequences can be interrupted by non-nucleotide components. The polynucleotide may be further modified after polymerization, for example being labeled by coupling to a labeling component. The term refers to both double-stranded and single-stranded polynucleotide molecules. Unless otherwise specified or required, any embodiment of polynucleotides in the present disclosure encompasses the double-stranded form and either of the two complementary single-stranded forms known or predicted to form the double-stranded form.

The term "encode", as being applied to polynucleotides, refers to a polynucleotide "encodes" a polypeptide, meaning that in its natural state or when manipulated by methods well known to those skilled in the art, the polynucleotide can produce the desired polypeptide and/or fragments thereof through transcription and/or translation, or produce RNA capable of encoding the desired polypeptide and/or fragments thereof. The antisense strand refers to a sequence complementary to such a polynucleotide, and the encoding sequence can be deduced therefrom.

The term "genomic DNA" denotes the DNA of the genome of an organism, including the DNA of the genomes of bacteria, archaea, fungi, protozoa, viruses, plants, or animals.

The term "manipulating" DNA includes binding to DNA, creating a nick in one strand, or cleaving two strands of DNA, or includes modifying or editing DNA or polypeptides that bind to DNA. Manipulation of DNA can silence, activate, or regulate the expression of RNA or polypeptides encoded by the DNA (to prevent transcription, reduce transcription activity, prevent translation, or lower translation level), or prevent or enhance the binding of polypeptides to DNA. Cleavage may be performed by a variety of methods, such as enzymatic or chemical hydrolysis of the phosphodiester bond. Single or double strands can be cleaved. DNA cleavage can lead to blunt or staggered ends.

The term "hybridizable", "complementary", or "substantially complementary" means that a nucleic acid (e.g., RNA) comprises a nucleotide sequence allowing it to non-covalently bind to another nucleic acid in a sequence-specific, antiparallel manner under appropriate in vitro and/or in vivo temperature and ionic strength conditions. This binding involves the formation of Watson-Crick base pairs and/or G/U base pairs, also known as "annealing" or "hybridization".

It is understood in the art that the sequence of the polynucleotide does not need to be 100% complementary to the sequence of the target nucleic acid to which it can specifically hybridize. Polynucleotides may hybridize on one or more segments. The polynucleotide may comprise at least 70%, at least 80%, at least 90%, at least 95%, at least 99%, or 100% sequence complementarity to the target region in the target nucleic acid sequence to which they target. Percentage complementarity between specific nucleic acid sequence segments can be routinely determined using known BLAST and PowerBLAST programs in the art.

The terms "peptide," "polypeptide," and "protein" are used interchangeably in the present disclosure and denote polymeric forms of amino acids in any length, which may include coded and non-coded amino acids, chemically or biochemically modified or derived amino acids, and polypeptides with modified peptide backbones.

The term "binding domain" refers to a protein domain that is capable of binding to another molecule non-covalently. The binding domains can bind to, for example, DNA molecules (DNA-binding proteins), RNA molecules (RNA-binding proteins), and/or protein molecules (protein-binding proteins). In the case of a protein domain-binding protein, it can bind to itself (to form homodimers, homotrimers, etc.), and/or it can bind to one or more molecules of one or more different proteins.

The term "conservative amino acid substitution" denotes the interchangeability of amino acid residues with similar side chains in a protein. Exemplary conservative amino acid substitution groups are: valine-leucine-isoleucine, phenylalanine-tyrosine, lysine-arginine, alanine-valine, and asparagine-glutamine.

The term a DNA sequence "encoding" a particular RNA is a DNA nucleic acid sequence that is transcribed into RNA. DNA polynucleotides can encode RNAs that are translated into proteins (mRNAs), or DNA polynucleotides can encode RNAs that are not translated into proteins (e.g., tRNAs, rRNAs, or gRNAs; (also known as "non-coding" RNAs or "ncRNAs")). "Protein encoding sequence" or a sequence encoding a specific protein or polypeptide is a nucleic acid sequence that is transcribed into mRNA (in the case of DNA) and translated (in the case of mRNA) into a polypeptide in vivo or in vitro under the control of an appropriate regulatory sequence.

The term "vector" or "expression vector" refers to a replicon, such as a plasmid, phage, virus, or cosmid, to which another DNA segment (i.e., an "insertion fragment") can be attached, allowing replication of the attached region in the cell.

The term "expression cassette" encompasses a DNA encoding sequence that is operably linked to a promoter. "Operably linked" indicates the components are connected in such a way that allows them to function as intended. The terms "recombinant expression vector" or "DNA construct" are used interchangeably in the present disclosure to denote a DNA molecule comprising a vector and at least one insertion fragment. Recombinant expression vectors are typically produced for the purpose of expressing and/or amplifying the insertion fragments or for constructing other recombinant nucleotide sequences.

When exogenous DNA, such as a recombinant expression vector, has been introduced into a cell, the cell is "genetically modified", "transformed", or "transfected" by the DNA. The presence of exogenous DNA results in permanent or transient genetic alterations. The transformed DNA may or may not be integrated into the genome of the cell.

The term "target DNA" is a DNA polynucleotide containing a "target site" or "target sequence". The terms "target site", "target sequence", "target protospacer DNA" or "protospacer-like sequence" are used interchangeably in the present disclosure to denote a nucleic acid sequence present in the target DNA, to which the DNA-targeting segment of the gRNA will bind if sufficient conditions for binding are present. RNA molecules contain sequences that bind to, hybridize with, or are complementary to target sequences in the target DNA, thereby directing the bound polypeptide to a specific position (target sequence) of the target DNA. "Cleavage" refers to the breakage of the covalent backbone of the DNA molecule.

The terms "nuclease" and "endonuclease" are used interchangeably to refer to enzymes having endonucleolytic degradation catalytic activity for polynucleotide cleavage. The "cleavage domain", "active domain", or "nuclease domain" of a nuclease is a polypeptide sequence or domain within a nuclease that has catalytic activity for DNA cleavage. The cleavage domain may be contained in a single polypeptide chain, or the cleavage activity may result from the conjugation of two or more polypeptides.

The term "localizing polypeptide" or "RNA-binding site-directed polypeptide" refers to a polypeptide that binds RNA and targets a specific DNA sequence.

The term "guide sequence" or DNA-targeting segment (or "DNA-targeting sequence") refers to a nucleotide sequence (the complementary strand of the target DNA) that is complementary to a specific sequence in the target DNA, referred to as a "protospacer-like" sequence in the context of the present disclosure.

The term "recombination" refers to the process of exchanging genetic information between two polynucleotides. As used in the present disclosure, "homology-directed repair (HDR)" denotes a specialized form of DNA repair that occurs, for example, during the repair of double-strand breaks in cells. This process, which requires nucleotide sequence homology, uses a "donor" molecule as a template for the repair of a "target" molecule (i.e., the molecule that undergoes the double-strand break) and results in the transfer of genetic information from the donor to the target. Homology-directed repair may cause alterations (e.g., insertions, deletions, mutations) in the sequence of the target molecule if the donor polynucleotide is different from the target molecule and some or all of the sequences of the donor polynucleotide are incorporated into the target DNA.

The term "non-homologous end joining (NHEJ)" refers to the repair of double-strand breaks in DNA by directly ligating the break ends to each other without the need for a homologous template. NHEJ frequently results in deletions of nucleotide sequences near double-strand break sites.

The term "treatment" includes preventing the onset of a disease or symptoms, suppressing a disease or symptoms, or alleviating a disease.

The terms "individual", "subject", "host", and "patient" are used interchangeably in the present disclosure and denote any mammalian subject, particularly humans, intended to be diagnosed, treated, or receive a therapy.

The present disclosure provides a small sized C2C9 nuclease for a CRISPR editing system, comprising: i) a RNA-binding portion that interacts with gRNA, and an active portion.

The C2C9 nuclease has at least one of the following activities: regulation of transcription of a target gene (e.g., target DNA), cleavage activity (endoribonuclease and/or endonuclease activity), gene editing activity, and the like. Gene editing includes, but is not limited to: gene cleavage, gene deletion, gene insertion, point mutation, transcription inhibition, transcription activation, base editing, and the like. The C2C9 nuclease may be derived from any biological species.

As a non-limiting illustrative example, the C2C9 nuclease can be used as a nuclease for, for example, a CRISPR editing system.

In the present disclosure, C2C9 nuclease variants can be obtained by modification, mutation, DNA shuffling, etc., and the C2C9 nuclease variants have improved desired characteristics, such as function, activity, kinetics, half-life, etc. The modifications can be, for example, deletions, insertions, or substitutions of amino acids, or for another example, replacement of the "cleavage domain" of the C2C9 nuclease with homologous or heterologous cleavage domains from different nucleases (e.g., the HNH domain of the CRISPR-associated nuclease). By any modification method for DNA-binding and/or DNA-modifying proteins known in the art, such as methylation, demethylation, acetylation, and the like, the DNA targeting specificity of C2C9 nuclease can be altered. DNA shuffling refers to exchanging sequence fragments between DNA sequences of C2C9 nucleases from different sources to produce chimeric DNA sequences encoding synthetic proteins with RNA-guided endonuclease activity. These modifications, mutations, DNA shuffling, etc. may be used singly or in combination.

The C2C9 nuclease comprises, from the N-terminus to the C-terminus:
a domain 1 including an amino acid sequence shown as SEQ ID NO. 1 or a variant having at least 30%, at least 35%, at least 40%, at least 45%, at least 50%, at least 55%, at least 60%, at least 65%, at least 70%, at least 75%, at least 80%, at least 85%, or at least 90% sequence identity to SEQ ID NO. 1;
and a domain 2, including an amino acid sequence shown as SEQ ID NO. 2 or a variant having at least 30%, at least 35%, at least 40%, at least 45%, at least 50%, at least 55%, at least 60%, at least 65%, at least 70%, at least 75%, at least 80%, at least 85%, or at least 90% sequence identity to SEQ ID NO. 2.

The C2C9 nuclease variant:
(I) has at least 20% (e.g., 30%, 40%, 50%, 60%, 70%, 80%, 85%, 90%, 91%, 92%, 93%, 94%, 95%, 96%, 97%, 98%, or 99%) sequence identity to the amino acid sequence of the wild-type C2C9 nuclease and retains the activity of the wild-type C2C9 nuclease.
(II) The C2C9 nuclease according to (I) or a variant thereof, further comprises other components, such as a nuclear localization signal fragment, to make the constructed C2C9 CRISPR system appropriately active in a cell-free, a prokaryotic cell, or a eukaryotic cell environment.
(III) Codon optimized variants of polynucleotide sequences encoding the wild-type C2C9 nuclease or variants according to (I) and (II).
(IV) Any variant according to the wild-type C2C9 nuclease, and (I) to (III), further comprises:
   (a) one or more modifications or mutations, that produce C2C9 with significantly reduced or undetectable nuclease activity; and
   (b) a polypeptide or domain with other functional activities.

Suitable polypeptides in (IV) (b) are linked to the C-terminal domain of the C2C9 nuclease or a variant thereof.

The C2C9 nuclease variant in (IV) can convert any base pair to any possible other base pair by modification or mutation, rather than causing a double-strand break of the target DNA sequence.

The C2C9 nuclease variant in (IV) may be a fusion or chimeric polypeptide formed from a wild-type C2C9 nuclease or a variant of (I) to (III) with a heterologous sequence. The heterologous sequence includes, but is not limited to, a light-induced transcriptional regulator, a small molecule/drug response transcriptional regulator, a transcription factor, a transcription repressor protein, and the like. In the formation of the fusion or chimeric polypeptide, the wild-type C2C9 nuclease or the variant of (I) to (III) may be a complete, or a partially or completely defective C2C9 nuclease. For example, a C2C9 nuclease containing an endonuclease domain with catalytic activity is fused to the Fokl domain to form a chimeric protein. Alternatively, after being modified, the C2C9 nuclease without endonuclease domain activity is fused to the Fokl domain to form a chimeric protein. Alternatively, epigenetic modifiers, tags, imaging agents, transcription regulators, histones, other components that regulate gene structure or activity, etc., are fused with the C2C9 nuclease to prepare chimeric proteins. In some embodiments, the heterologous sequence may provide tags for easy tracking or purification, for example, fluorescent proteins such as green fluorescent protein (GFP), YFP, RFP, CFP, etc.; His tag; Hemagglutinin (HA) tag; FLAG tag; Myc tag, etc. In some embodiments, the heterologous sequence may provide subcellular localization for the C2C9 nuclease.

In some embodiments, the C2C9 nuclease variant is a modified form of C2C9 nuclease. In some embodiments, the modified form of C2C9 nuclease comprises an amino acid change that reduces the naturally occurring nuclease activity of C2C9 nuclease. For example, in some embodiments, the modified form of the C2C9 nuclease has less than 50%, less than 40%, less than 30%, less than 20%, less than 10%, less than 5%, or less than 1% of the nuclease activity of the corresponding wild-type C2C9 nuclease. In some embodiments, the modified form of C2C9 nuclease has no significant nuclease activity, but retains the ability to interact with gRNA. In some embodiments, the modified form of C2C9 nuclease has no nuclease activity. In some embodiments, the modified form of the C2C9 nuclease includes a mutant polypeptide having altered or no DNA endonuclease activity, when its endoribonuclease activity or binding affinity to DNA is not considerably reduced or increased.

In some embodiments, the C2C9 may be used in combination with other enzyme compositions or other compositions to further develop various potential applications of the C2C9 nuclease. The application example of the C2C9 nuclease variant in (IV) includes, but is not limited to, for example, a C2C9 nuclease based-single base editing system obtained from the fusion of inactivated C2C9 and base deaminase; a C2C9 nuclease based-Prime editing system obtained from the fusion of inactivated C2C9 and reverse transcriptase; a C2C9 nuclease based-transcription activation system obtained from the fusion of inactivated C2C9 and transcription activators; a C2C9 nuclease based- epigenetic modification system obtained from the fusion of inactivated C2C9 and nucleic acid epigenetic modifying enzymes; a C2C9 nuclease based-transcription inhibition system using inactivated C2C9.

The C2C9 nuclease variants may have, but not limited to, the following specific properties :
an enhanced or reduced ability to bind to the target site, or a retained ability to bind to the target site;
an enhanced or reduced endoribonuclease and/or endonuclease activity, or a retained endoribonuclease and/or endonuclease activity;
a deaminase activity, which acts on cytosine, guanine, or adenine bases and subsequently, through deaminated site replication and intracellular repair to produce guanine, thymine, and guanine, respectively;
an activity that regulates the transcription of target DNA, which can either increase or decrease the transcription of target DNA at particular positions in the target DNA;
an altered DNA targeting specificity;
an increased, decreased, or maintained stability;
an ability to cleave the complementary strand of target DNA, but with a reduced ability to cleave the non-complementary strand of target DNA;
an ability to cleave the non-complementary strand of target DNA, but with a reduced ability to cleave the complementary strand of target DNA;
a reduced ability to cleave both the complementary and non-complementary strands of target DNA;
an enzyme activity that modifies a DNA-associated polypeptide (e.g., a histone), the enzyme activity may be one or more of a methyltransferase activity, a demethylase activity, an acetyltransferase activity, a deacetylase activity, a kinase activity, a phosphatase activity, a ubiquitin ligase activity, a deubiquitinase activity, a ribosylation activity, and the like (the enzyme activity catalyzes the covalent modification of the protein; for example, C2C9 nuclease variants modify histones by methylation, acetylation, ubiquitination, phosphorylation, etc., to induce structural changes in the histone-associated DNA, thereby controlling the structure and properties of DNA.)

In some embodiments, the C2C9 nuclease variant has no cleavage activity. In some embodiments, the C2C9 nuclease variant has single-stranded cleavage activity. In some embodiments, the C2C9 nuclease variant has double-stranded cleavage activity.

Having enhanced activity or ability means having an activity or ability that is elevated by at least 1%, 5%, 10%, 20%, 30%, 40%, or 50% compared to wild-type C2C9 nuclease.

Having reduced activity and ability means having less than 50%, less than 40%, less than 30%, less than 20%, less than 10%, less than 5%, or less than 1% activity or ability compared to wild-type C2C9 nuclease.

In the present disclosure, the C2C9 nuclease is preferably selected from the group consisting of SEQ ID NOs. 3-117 and the like.

In some embodiments, the various enzymatic properties of different C2C9 nucleases (e.g., different PAM sequence preferences) are studied, C2C9 nucleases from different specie sources are beneficial for providing different applications, e.g., increasing or decreasing enzymatic activity; enhancing or lowering cytotoxicity levels; altering NHEJ, performing homology directed repair, balancing single-strand break and double-strand break, etc.

In certain embodiments, the C2C9 nuclease is derived from *Actinomadura craniellae.* The C2C9 nuclease comprises an amino acid sequence shown as SEQ ID NO.3 or a variant having at least about 90% (e.g., at least about any of 91%, 92%, 93%, 94%, 95%, 96%, 97%, 98%, 99%, and more) sequence identity to SEQ ID NO.3. *Actinomadura craniellae* C2C9 or "AcC2C9" recognizes the PAM sequence AAN. In some embodiments, the C2C9 nuclease is derived from *Corynebacterium glutamicum* (SEQ ID NO.5 ) , *Corynebacterium glutamicum* C2C9 or "CgC2C9" recognizes the PAM sequence AAN. In certain embodiments, the C2C9 nuclease is derived from *Rothia dentocariosa* (SEQ ID NO.20), *Rothia dentocariosa* C2C9 or "RdC2C9" recognizes the PAM sequence AAG. In some embodiments, the RdC2C9 nuclease recognizes the PAM sequence AAA, AAT, or AAC. In some embodiments, the C2C9 is derived from *Micrococcus luteus* (SEQ ID NO. 12), *Micrococcus luteus* C2C9 or "MiC2C9" recognizes the PAM sequence AAN. The AAN comprises AAA, AAC, AAG, and AAT.

C2C9 nucleases from different species or different C2C9 nucleases from the same species may require different PAM sequences in the target DNA, and thus, for the particularly selected C2C9 nuclease, the PAM sequence requirements may be different from those of the foregoing PAM sequences.

In some embodiments, these small sized C2C9s may be used in any of the systems, compositions, kits, and methods described below in the present disclosure.

In a preferred embodiment, the C2C9 nuclease is derived from *Actinomadura craniellae* (AcC2C9), and the C2C9 nuclease has a sequence shown as SEQ ID NO. 3.

In some embodiments, the AcC2C9 nuclease variant:
(I) has at least 20% (e.g., 30%, 40%, 50%, 60%, 70%, 80%, 85%, 90%, 95%, or 99%) sequence identity to the amino acid sequence of SEQ ID NO. 3 which corresponds to the wild-type AcC2C9 nuclease, and has increased, decreased, or maintained activity compared to the wild-type C2C9 nuclease.
(II) The AcC2C9 nuclease according to (I) or a variant thereof, further comprises other components, such as a nuclear localization signal fragment, to make the constructed C2C9 CRISPR system appropriately active in a cell-free, a prokaryotic cell, or a eukaryotic cell environment.
(III) Codon optimized variant of polynucleotide sequences encoding the SEQ ID NO. 3 corresponding to wild-type AcC2C9 nuclease or variants according to (I) and (II).
(IV) Any variant according to SEQ ID NO. 3 corresponding to the wild-type AcC2C9 nuclease and (I) to (III), further comprises:
   (a) one or more modifications or mutations, that produce C2C9 with significantly reduced or undetectable nuclease activity; and
   (b) a polypeptide with other functional activities.

The C2C9 nuclease provided by the present disclosure has a small number of amino acids. In a preferred embodiment, when the C2C9 nuclease has an amino acid sequence shown as SEQ ID NO.3, it interacts with the PAM sequence AAN. The cleavage site is usually located within one to three base pairs upstream of the PAM sequence. C2C9 nucleases from different species or different C2C9 nucleases from the same species may require different PAM sequences in the target DNA, and thus, for the particularly selected C2C9 nuclease, the PAM sequence requirements may be different from those of the foregoing PAM sequences. C2C9 can be engineered to target the PAM sequence "AAN" or other suitable PAM sequences.

In some embodiments, the AcC2C9 nuclease variant has at least 90% (e.g., at least about 91%, 92%, 93%, 94%, 95%, 96%, 97%, 98%, or 99%) sequence identity to the amino acid sequence shown as SEQ ID NO.3 and maintains the activity of the AcC2C9 nuclease.

If not otherwise specified, the terms "C2C9" and "C2C9 nuclease" include wild-type C2C9 nuclease as well as all variants thereof, and one skilled in the art can determine the type of C2C9 nuclease variant by conventional means without being limited to those exemplified above.

The present disclosure also provides a nucleic acid comprising a nucleotide encoding an AcC2C9 nuclease. In one embodiment, the present disclosure provides codon-optimized polynucleotide sequences encoding one or more functional domains of C2C9 or encoding polypeptides having the same functions as the polypeptide encoded by the original natural nucleotide sequence. One skilled in the art may perform codon optimization of a nucleotide sequence or recombinant nucleic acid according to the particular target species in which the aforementioned C2C9 nuclease is used. Codon optimization can be carried out by other methods known in the art, the codon-optimized polynucleotide sequences have less than 100% (e.g., less than 80%, 81%, 82%, 83%, 84%, 85%, 86%, 87%, 88%, 89%, 90%, 91%, 92%, 93%, 94%, 95%, 96%, 97%, 98% or 99%) identity to the natural nucleotide sequence shown as SEQ ID NO.121. The polynucleotides of the present disclosure are codon-optimized to increase the expression of the encoded C2C9 nuclease in target cells. In certain embodiments, the polynucleotides of the present disclosure are codon-optimized to increase expression in human cells. In certain embodiments, the polynucleotides of the present disclosure are codon-optimized to increase expression in E. coli cells. In some embodiments, the polynucleotides of the present disclosure are codon-optimized to increase expression in fungal cells. In certain embodiments, the polynucleotides of the present disclosure are codon-optimized to increase expression in insect cells.

In one embodiment, the nucleic acid encoding the AcC2C9 nuclease has a sequence shown as SEQ ID NO. 121. In one embodiment, the present invention provides codon-optimized polynucleotide sequences of AcC2C9 nuclease having at least 90%, 92%, 93%, 94%, 95%, 96%, 97%, 98%, 99%, 99.2%, 99.5%, 99.8%, 99.9%, or 100% sequence identity to SEQ ID NO.121. In a preferred embodiment, an encoding sequence of the AcC2C9 nuclease, which has been optimized based on human codons, is shown as SEQ ID NO. 122, which encodes one or more functional domains of C2C9 or encodes a polypeptide having the same functions as the polypeptide encoded by the original natural nucleotide sequence. In some embodiments, the polynucleotide encoding the C2C9 nuclease has at least about 90% (e.g., at least any of about 91%, 92%, 93%, 94%, 95%, 96%, 97%, 98%, 99%, or more) sequence identity to SEQ ID NOs.121-122.

In some embodiments, the nucleic acid encoding the C2C9 nuclease is DNA. In some embodiments, the nucleic acid encoding the C2C9 nuclease is RNA. In some embodiments, the nucleic acid encoding the C2C9 nuclease is an expression vector, such as a recombinant expression vector. Any suitable expression vector may be used as long as it is compatible with the host cell, including, but not limited to, viral vectors (e.g., poxvirus-based viral vectors; poliovirus; adenovirus; adeno-associated virus; SV40; herpes simplex virus; human immunodeficiency virus), retroviral vectors (e.g., murine leukemia virus, spleen necrosis virus, and vectors derived from retroviruses, such as Rous sarcoma virus, Harvey sarcoma virus, avian leukosis virus, lentiviruses, human immunodeficiency virus, myeloproliferative sarcoma virus, and mammary tumor viruses), etc.

In some embodiments, the nucleotide sequence encoding the C2C9 nuclease is operably linked to a control element, for example, a transcription control element (such as a promoter). In some embodiments, the nucleotide sequence encoding the C2C9 nuclease is operably linked to an inducible promoter. In some embodiments, the nucleotide sequence encoding the C2C9 nuclease is operably linked to a constitutive promoter. Transcription control elements can function in eukaryotic cells, such as mammalian cells; or prokaryotic cells (e.g., bacterial or archaeal cells). In some embodiments, the nucleotide sequence encoding the C2C9 nuclease is operably linked to multiple control elements that allow expression of the nucleotide sequence encoding the C2C9 nuclease in both prokaryotic and eukaryotic cells. In some embodiments, the polynucleotide sequence encoding the C2C9 nuclease is operably linked to a suitable nuclear localization signal fragment that is for expression in a cellular or in vitro environment.

In the present disclosure, the polynucleotide encoding the C2C9 nuclease can be, for example, chemically synthesized, thereby the polynucleotide can be easily modified. The modification may be any modification known in the art. In some embodiments, the polynucleotide encoding the C2C9 nuclease comprises one or more modifications, a number of modifications can be readily incorporated to enhance transcription activity, to alter enzyme activity, to improve its translation, stability (e.g., to increase its resistance to protein hydrolysis, degradation), or specificity, to alter solubility, to alter delivery, and to reduce innate immune response in a host cell. The modification may be any modification known in the art. In some embodiments, DNA or RNA encoding the C2C9 nuclease, that is introduced into a cell, is modified to edit the locus of any one or more genomes. In some embodiments, the nucleic acid sequence encoding the C2C9 nuclease is modified, e.g., codon-optimized. The modification may be a single modification, or a combination of modifications.

In the present disclosure, the nucleic acid comprising the polynucleotide encoding the C2C9 nuclease may be a nucleic acid analog. Examples include polynucleotide analogs and peptide nucleic acids with excellent hybridization performances.

In the present disclosure, the C2C9 nuclease, or polynucleotide encoding the C2C9 nuclease, is suitable for any organism or in vitro environment, including, but not limited to, bacteria, archaea, fungi, protozoa, plants, or animals. Accordingly, applicable target cells include, but are not limited to, eukaryotic and prokaryotic cells, for example, bacterial cells, archaeal cells, fungal cells, protozoan cells, plant cells, or animal cells; The eukaryotic cells comprise mammalian cells and plant cells, and the prokaryotic cells comprise Escherichia coli and Klebsiella pneumoniae. Applicable target cells can be any type of cell, including stem cells, somatic cells, etc. The cells may be in vivo or ex vivo. In some embodiments, the C2C9 nuclease or the nucleic acid encoding the C2C9 nuclease is formulated in liposomes or lipid nanoparticles.

The present disclosure also provides a use of the C2C9 nuclease in the construction of a CRISPR/C2C9 gene editing system.

The present disclosure also provides suitable PAM sequences for in vivo, isolated cells, or in vitro environments, the PAM is AAN and/or GAN.

The present disclosure also provides suitable PAM sequences and suitable guides for prokaryotic, eukaryotic, and in vitro environments. The present disclosure provides a guide RNA (gRNA) that directs a nuclease, such as a C2C9 nuclease, to a specific target sequence in a target gene.

In some embodiments, the gRNA comprises:
a first fragment, that is a nucleotide sequence complementary to a target sequence in a target gene (also called "gene targeting sequence" or "gene targeting fragment"); and
a second fragment that interacts with the C2C9 nuclease (also called "protein-binding sequence" or "protein-binding fragment").

In some embodiments, the gRNA comprises a repeat-spacer region array, wherein the spacer region comprises nucleic acid sequences complementary to the target sequence in the gene.

In some embodiments, the gRNA comprises:
i. a gene-targeting segment (e.g., DNA-targeting segment) that is capable of hybridizing to a target sequence;
ii. a tracr pairing sequence, and
iii. a tracr RNA sequence.

The guide RNA is a strand that is formed by sequentially linked DNA-targeting segment (i), the tracr pairing sequence (ii), and the tracr RNA sequence (iii). The guide RNA may comprise two strands, wherein one strand is formed by linked gene-targeting segment (i) and the tracr pairing sequence (ii), and the other strand is the tracr RNA sequence (iii).

The gene-targeting segment (i) is preferably a RNA sequence corresponding to a nucleic acid fragment of 20 bp in length following the PAM sequence.

The tracr pairing sequence (ii) hybridizes with the tracr RNA sequence (iii) and forms a stem-loop structure.

The tracr pairing sequence (ii) and the tracr RNA sequence (iii) can be linked together to form a single guide RNA backbone sequence.

The RNA sequence (crRNA) obtained by ligating the gene-targeting segment (i) hybridized to the target sequence and the tracr pairing sequence (ii), together with the tracr RNA sequence (iii), act as two separate RNA sequences, that, in their simultaneous presence, can mediate C2C9 nucleic acid endonuclease activity. The complete guide RNA expression construct against the target sequence obtained by ligating the guide RNA backbone sequence and the DNA-targeting segment (i) hybridized to the target sequence can similarly mediate C2C9 nucleic acid endonuclease activity.

The gene-targeting segment of a gRNA contains a nucleotide sequence that is complementary to a sequence in the target gene, and this gene-targeting sequence interacts with the target gene by hybridization (i.e., base pairing) in a sequence-specific manner. The gene targeting sequence of the gRNA can be modified, for example, by genetic engineering, to allow the gRNA to hybridize to any desired sequence in the target gene. The gRNA directs the bound polypeptide to a specific nucleotide sequence with the target gene via the gene-targeting sequence described above.

The stem-loop structure forms a protein-binding structure that interacts with the C2C9 nuclease. In some embodiments, the protein-binding structure of the gRNA comprises 4 stem-loop structures, and the tracr pairing sequence (ii) is typically paired with the tracr RNA sequence (iii) by base complementarity. The base sequence of the gRNA can be modified to increase C2C9 nuclease activity or reduce non-specific recognition.

In some embodiments, the target gene is a DNA sequence. In some embodiments, the target gene is a RNA sequence.

In some embodiments, the gRNA is sgRNA. In a preferred embodiment, the gRNA includes a sequence shown as SEQ ID NO. 123.

In some embodiments, the targeting sequence of the target gene may have a length of 12-40 nucleotides, for example, may be 13-20, 18-25, 22-32, 26-37, 30-38, or 32-40 nucleotides in length. The percentage of complementarity between the targeting segment (i) of the guide RNA and the target sequence of the target gene may be at least 50% (e.g., at least 55%, at least 60%, at least 65%, at least 70%, at least 75%, at least 80%, at least 85%, at least 90%, at least 95%, at least 97%, at least 98%, at least 99%, or 100%).

In some embodiments, the gRNA also includes a transcription terminator.

The present disclosure also provides a modified gRNA, which can hybridize to any desired sequence in a target gene. Alternatively, the characteristics of the gRNA are altered by modification, such as enhancing the stability of the gRNA by modification, including, but not limited to, prolonging its half-life in the cell by increasing its resistance to degradation of ribonuclease (RNase) in the cell. Alternatively, it can be used to enhance the formation or stability of the CRISPR-C2C9 genome editing complex comprising the gRNA and the endonuclease (e.g., C2C9 nuclease) after modification. Alternatively, it can be used to enhance the specificity of the genome editing complex after modification. Alternatively, it can be used to enhance the initiation site, stability, or kinetics of the interaction between the genome editing complex and the target sequence in the genome after modification. Alternatively, it can be used after modification to reduce the possibility or extent of an innate immune response caused by introducing RNA into the cell, etc. In the present disclosure, a variety of properties of the CRISPR-C2C9 system (described below) can be altered by modification of the gRNA, such as enhancing the formation, target activity, specificity, stability, or kinetic characteristics of the CRISPR-C2C9 genome editing complex. The RNA can be modified using methods known in the art, including, but not limited to, 2'-fluorine or 2'-amino modification on the ribose of the pyrimidine, base residues, or the reverse bases at the 3' end of the RNA. In the present disclosure, any one modification or combination of modifications may be applied to the gRNA. In some embodiments, the modification of sgRNA introduced into the cell allows the edition of the locus of any one or more genomes.

The present disclosure provides the nucleic acid comprising the nucleotide sequence encoding the gRNA. In some embodiments, the nucleic acid encoding the gRNA is an expression vector, such as a recombinant expression vector. Any suitable expression vector may be used as long as it is compatible with the host cell, including, but not limited to, viral vectors (e.g., poxvirus-based viral vectors; poliovirus; adenovirus; adeno-associated virus; SV40; herpes simplex virus; human immunodeficiency virus), retroviral vectors (e.g., murine leukemia virus, spleen necrosis virus, and vectors derived from retroviruses, such as Rous sarcoma virus, Harvey sarcoma virus, avian leukosis virus, lentiviruses, human immunodeficiency virus, myeloproliferative sarcoma virus, and mammary tumor viruses), etc.

In some embodiments, multiple gRNAs are used in the same cell to simultaneously regulate transcription at different positions on the same target gene or different target genes. When multiple gRNAs are used at the same time, they can be present on the same expression vector or on different vectors, or they can be expressed simultaneously. When present on the same vector, they can be expressed under the same control element.

In some embodiments, the nucleotide sequence encoding the gRNA is operably linked to the control element, such as a transcription control element (e.g., a promoter). In some embodiments, the nucleotide sequence encoding the gRNA is operably linked to the inducible promoter. In some embodiments, the nucleotide sequence encoding the gRNA is operably linked to the constitutive promoter. Transcription control elements can function in eukaryotic cells, such as mammalian cells, or prokaryotic cells (e.g., bacteria or archaeal cells). In some embodiments, the nucleotide sequence encoding the gRNA is operably linked to multiple control elements, allowing expression of the nucleotide sequence encoding the gRNA in both prokaryotic and eukaryotic cells.

In the present disclosure, the gRNA can be synthesized, e.g., by chemical methods, thus the gRNA can be easily modified. The modifications can be made by any methods known in the art, e.g., using polyA tails, adding 5' cap analogs, 5' or 3' untranslated regions (UTRs), including thiophosphorylated 2'-O-methyl nucleotides at the 5' or 3' end, or treating with phosphatase to remove the 5' end phosphates, etc.

In some embodiments, the nucleotide sequence encoding the gRNA comprises one or more modifications that may be used, for example, to enhance activity, stability, or specificity, to alter delivery, to reduce an innate immune response in a host cell, or for other enhancements.

In some embodiments, one or more targeting portions or conjugates that enhance the activity, cellular distribution, or cellular uptake of the nucleotide sequence encoding the gRNA are chemically linked to the gRNA. The targeting portions or conjugates may comprise conjugate groups covalently bound to the functional groups. The conjugate groups include reporter molecules, polyamine, and polyethylene glycol. In some embodiments, a cluster that enhances the pharmacodynamics is linked to the gRNA, and the cluster that enhances the pharmacodynamics comprises a cluster that improves uptake, enhances resistance to degradation, and/or enhances sequence-specific hybridization to a target nucleic acid.

In the present disclosure, the nucleic acid comprising the polynucleotide encoding the gRNA may be a nucleic acid analog. Examples include polynucleotide analog or peptide nucleic acid with excellent hybridization properties.

In the present disclosure, the gRNA, or the polynucleotide encoding the gRNA is suitable for any biological or in vitro environment, including, but not limited to, bacteria, archaea, fungi, protozoa, plants, or animals. Accordingly, applicable target cells include, but are not limited to, bacteria cells, archaeal cells, fungal cells, protozoan cells, plant cells, or animal cells. Applicable target cells can be any type of cell, including stem cells, somatic cells, etc.

The present disclosure also provides a recombinant expression vector comprising (i) the nucleotide sequence encoding the gRNA, and (ii) the nucleotide sequence encoding the C2C9 nuclease. In one embodiment, the regulated transcription site in the target gene is determined by the gRNA.

The present disclosure also provides a CRISPR-C2C9 system based on the C2C9 nuclease, the system comprises (a) the C2C9 nuclease as described above or the above mentioned nucleic acid comprising the polynucleotide encoding the C2C9 nuclease; and/or, (b) one or more gRNAs or the nucleic acid encoding the gRNA as described above. The gRNA is capable of directing the C2C9 nuclease to the target sequence.

In some embodiments, the C2C9 nuclease is directly provided as a protein, for example, using exogenous protein for protoplast transfection and/or nucleic acid transformation of fungi. The C2C9 nuclease can be introduced into the cell by any suitable method, such as by injection.

In the system described herein, the C2C9 nuclease and gRNA can form a complex in the host cell, to recognize the PAM sequence on the target gene (e.g., target DNA) sequence. The target sequence of the CRISPR/C2C9 gene editing system is a nucleic acid fragment (e.g., a DNA fragment) of 20 bp in length following the PAM sequence. In one embodiment, the complex can selectively regulate the transcription of target DNA in the host cell. The CRISPR/C2C9 gene editing system is able to cleave the double strand of the target DNA, causing DNA breaks. The major cleavage site on the targeting strand (a single strand of DNA that is complementary to gRNA, also known as the Targeting strand or TS strand) is 21-22 nt downstream of the 3' end of the PAM site, whereas the major cleavage site on the non-targeting strand (a single strand of DNA that is not complementary to gRNA, also known as the Non-targeting strand or NTS strand) is 15-16 nt downstream of the 3' end of the PAM site.

In one embodiment, the system comprises the gRNA and the C2C9 nuclease complex.

In one embodiment, the system comprises the recombinant expression vector. In one embodiment, the system comprises the recombinant expression vector, and the recombinant expression vector comprises (i) the nucleotide sequence encoding the gRNA, wherein the gRNA comprises (a) the first segment of the nucleotide sequence complementary to the sequence in the target DNA; and (b) the second segment interacting with C2C9 nuclease; and (ii) the nucleotide sequence encoding the C2C9 nuclease, wherein the C2C9 nuclease comprises:(a) the RNA-binding portion that interacts with the gRNA; and (b) the active portion regulating the transcription in the target DNA. The regulated site of the transcription in the target DNA is determined by the gRNA.

In certain embodiments, the system comprises the C2C9 nuclease shown as SEQ ID NO.3. In certain embodiments, the system comprises the nucleic acid encoding the C2C9 nuclease, and the nucleic acid has at least about 90% (e.g., at least about 91%, 92%, 93%, 94%, 95%, 96%, 97%, 98%, or 99%) sequence identity to SEQ ID NO. 121 or SEQ ID NO. 122. In certain embodiments, the system comprises the nucleic acid encoding the C2C9 nuclease, and the nucleic acid comprises (or consists of) the sequence shown as SEQ ID NO. 121 or SEQ ID NO. 122. In some embodiments, the gRNA in the system is a single gRNA (sgRNA). In some embodiments, the system further comprises one or more additional gRNAs or the nucleic acids encoding one or more additional gRNAs.

In some embodiments, the system contains a suitable promoter and/or a suitable nuclear localization signal fragment for expression in a cellular or in vitro environment. The polynucleotide sequence encoding the C2C9 nuclease is operably linked to (i) the suitable promoter for expression in a cellular or in vitro environment; and/or (ii) the suitable nuclear localization signal fragment.

In some embodiments, the polynucleotide sequence encoding the gRNA in the system is operably linked to (i) the suitable promoter for expression in a cellular or in vitro environment; and/or (ii) the suitable nuclear localization signal fragment.

In certain embodiments, the present disclosure provides the system, comprising (a) the C2C9 nuclease, wherein the C2C9 nuclease comprises the amino acid sequence of SEQ ID NO.3 or a variant thereof having at least 90% sequence identity to SEQ ID NO.3; and (b) the gRNA, wherein the gRNA is capable of directing the C2C9 nuclease or a variant thereof to the target polynucleotide sequence.

In certain embodiments, the present disclosure provides the system, comprising (a) the C2C9 nuclease or the nucleic acid encoding the C2C9 nuclease, wherein the C2C9 nuclease comprises the amino acid sequence of SEQ ID NO.3 or a variant thereof having at least 90% sequence identity to SEQ ID NO.3, the nucleic acid encoding the C2C9 nuclease comprises the polynucleotide sequence of any one of SEQ ID NO. 121 and SEQ ID NO. 122 or a variant thereof having at least 90% sequence identity to the polynucleotide sequence of any one of SEQ ID NO. 121 and SEQ ID NO. 122; and (b) the gRNA or the nucleic acid encoding the gRNA, wherein the gRNA is capable of directing the C2C9 nuclease or a variant thereof to the target polynucleotide sequence. In some embodiments, the gRNA is sgRNA.

The system described herein may comprise a single gRNA or multiple gRNAs at the same time. In one embodiment, the system comprises multiple gRNAs at the same time to simultaneously modify different positions on the same target DNA or different target DNAs. In one embodiment, the two or more guide RNAs target the same gene, transcript, or locus. In one embodiment, the two or more guide RNAs target different unrelated loci. In some embodiments, the two or more guide RNAs target different but related loci.

The components of the system described herein may be transported by a carrier. For example, for polynucleotides, possible methods may include, but are not limited to, nanoparticles, liposomes, ribonucleoproteins, small molecule RNA-conjugates, chimeras, RNA-fusion protein complexes, etc.

In some embodiments, the C2C9 or gRNA may be constructed in a vector, wherein the nucleotide sequence encoding the C2C9 and/or gRNA is operably linked to a control element, such as a transcription control element (e.g., a promoter). Transcription control elements can function in eukaryotic cells (e.g., mammalian cells) or prokaryotic cells (e.g., bacteria or archaeal cells). In some embodiments, the nucleotide sequence encoding the gRNA and/or the C2C9 nuclease is operably linked to multiple control elements, allowing expression of the nucleotide sequence encoding the guide RNA and/or the specifically modified polypeptide in two prokaryotes. In some embodiments, the nucleotide sequences encoding gRNA and/or C2C9 nuclease are constructed on the same vector. In some embodiments, the nucleotide sequences encoding gRNA and/or C2C9 nuclease are constructed on different vectors. In some embodiments, the nucleotide sequence encoding gRNA and/or C2C9 nuclease is operably linked to the constitutive promoter. In some embodiments, the nucleotide sequence encoding gRNA and/or C2C9 nuclease is operably linked to the inducible promoter.

The system described herein may further comprise one or more donor templates. In some embodiments, the donor template comprises a donor sequence for insertion into the target gene. In some embodiments, the donor template comprises a donor box that has gRNA target sites flanked on one or both sides. In some embodiments, the donor box of the donor template is flanked on both sides by gRNA target sites, wherein the gRNA target sites of the donor template are an inverse complementary sequence to genomic gRNA target sites of the gRNA in the system. The donor sequence is typically different from the genomic sequence it displaces, i.e., the donor sequence typically contains base changes, insertions, deletions, inversions, or rearrangements relative to the genomic sequence while maintaining sufficient homology to support homology-directed repair. The donor sequence may be single-stranded DNA, single-stranded RNA, double-stranded DNA, or double-stranded RNA. In some embodiments, the donor sequence is an exogenous sequence. In some embodiments, the donor sequence is a donor DNA sequence (including donor single-stranded or double-stranded DNA), wherein the target DNA sequence is edited by homology-directed repair. In some embodiments, the donor template is physically linked to the gRNA in the system. In some embodiments, a complete, or a partially or fully defective C2C9 nuclease, or a gRNA is ligated to the donor template, and directed by one or more gRNA molecules to to one or more specific target gene (e.g., target DNA) sites, facilitating homologous recombination of exogenous DNA sequences.

The systems described herein may further comprise a dimeric FOK1 nuclease, a complete, or a partially or fully defective C2C9 nuclease, or a gRNA is ligated to the dimeric FOK1 nuclease to guide endonuclease cleavage when the dimeric FOK1 nuclease is directed to one or more specific DNA target sites by one or more gRNA molecules.

The system described herein can edit or modify DNA at multiple locations in a cell for gene therapy, including, but not limited to, gene therapy for disease, biological research, crop resistance improvement or yield enhancement, and the like.

In the present disclosure, the system is suitable for any biological or in vitro environment, including, but not limited to, bacteria, archaea, fungi, protozoa, plants, or animals. Accordingly, applicable target cells include, but are not limited to, bacterial cells, archaeal cells, fungal cells, protozoan cells, plant cells, or animal cells. Applicable target cells can be any type of cell, including stem cells, somatic cells, etc. The cells may be in vivo or isolated.

The present disclosure also provides a composition, comprising one or more of the aforementioned C2C9 nuclease or the polynucleotide encoding it, the gRNA or the polynucleotide encoding it, the recombinant expression vector, and the system. The composition may also comprise acceptable vehicles, media, and the like. The acceptable vehicles and media are for example sterile water or saline, stabilizers, excipients, antioxidants (ascorbic acid, etc.), buffers (phosphoric acid, citric acid, other organic acids, etc.), preservatives, surfactants (PEG, Tween, etc.), chelating agents (EDTA, etc.), binders, and the like. Also, other low molecular weight peptides, proteins such as serum albumin, gelatin, or immunoglobulin; amino acids such as glycine, glutamine, asparagine, arginine, and lysine; sugars such as polysaccharides and monosaccharides, or carbohydrates; sugar alcohols such as mannitol or sorbitol may be included. When preparing aqueous solutions for injection, such as saline, or isotonic solutions containing glucose or other adjuvants (such as D-sorbitol, D-mannose, D-mannitol, sodium chloride), appropriate solubilizers such as alcohols (ethanol, etc.), polyols (propylene glycol, PEG, etc.), nonionic surfactants (Tween 80, HCO-50), etc., may be used. In some embodiments, the composition comprises the gRNA and a buffer for stabilizing the nucleic acid.

The present disclosure also provides a kit, comprising the system or composition as described above. The kit may further comprise one or more of, e.g. dilution buffer, wash buffer, control reagent, etc. In some embodiments, the kit comprises (a) the C2C9 nuclease or the nucleic acid encoding the C2C9 nuclease as described above; and (b) the gRNA or the nucleic acid encoding the gRNA, wherein the gRNA is capable of directing the C2C9 nuclease or a variant thereof to the target polynucleotide sequence. In some embodiments, the kit further comprises the donor template including a heterologous polynucleotide sequence, wherein the heterologous polynucleotide sequence is capable of being inserted into the target polynucleotide sequence.

The present disclosure provides a use of the C2C9 nucleases or the polynucleotide encoding it, the gRNA or the polynucleotide encoding it, the recombinant expression vector, the system, the composition, and the kit described above in any of in vivo, in isolated cells, or in a cell-free system, including but not limited to:
cleaving a target gene;
manipulating the expression of the target gene;
genetically modifying the target gene;
genetically modifying a target gene-related peptide;
intentionally and controllably performing damage at any desired location of the target gene;
intentionally and controllably performing repair at any desired location of the target gene; and
modifying the target genes by means other than introducing double-strand breaks (the C2C9 nuclease is enzymatically active and it modifies the target gene by means other than introducing double-strand breaks; the enzymatic activity may be inherent to C2C9 itself, or obtained by, for example, fusing a heterologous polypeptide having an enzymatic activity to the C2C9 nuclease to form a chimeric C2C9 nuclease, and the enzymatic activity includes, but is not limited to, methyltransferase activity, deamidation activity, dismutase activity, alkylation activity, demethylase activity, DNA repair activity, transposase activity, recombinase activity, DNA damage activity, depurination activity, oxidation activity, pyrimidine dimer formation activity, and the like).

In some embodiments, the target gene is a target DNA. In some embodiments, the target gene is a target RNA.

In the present disclosure, the C2C9 can be used in combination with other enzyme components or other components, to further develop various potential applications of C2C9 nuclease. The application examples include, but are not limited to, a C2C9 nuclease based-single base editing system obtained from the fusion of inactivated C2C9 and base deaminase; a C2C9 nuclease based-Prime editing system obtained from the fusion of inactivated C2C9 and reverse transcriptase; a C2C9 nuclease based-transcription activation system obtained from the fusion of inactivated C2C9 and transcription activator; a C2C9 nuclease based-epigenetic modification system obtained from the fusion of inactivated C2C9 and nucleic acid epigenetic modifying enzymes; a C2C9 nuclease based-transcription inhibition system using inactivated C2C9.

The C2C9 nuclease or the polynucleotide encoding it, the gRNA or the polynucleotide encoding it, the recombinant expression vector, the system, the composition, and the kit of the present disclosure can be applied in the field of research, in the field of diagnostics, in the field of industry (e.g., microbial engineering), in the drug discovery (e.g., high-throughput screening), in the target validation, in the field of imaging, and in the field of therapeutics, etc. In the research field, they can be used to, for example, determine the effect of enhancing the transcription regulation of target nucleic acids, development, growth, metabolism (e.g., precise control and regulation of biosynthetic pathways by controlling the levels of specific enzymes), gene expression, and the like.

In the present disclosure, the C2C9 nuclease or the polynucleotide encoding it, the gRNA or the polynucleotide encoding it, the recombinant expression vector, the system, the composition, and the kit, are suitable for any organism, including, but not limited to, bacteria, archaea, fungi, protozoa, plants, or animals. Accordingly, applicable target cells include, but are not limited to, bacterial cells, archaeal cells, fungal cells, protozoan cells, plant cells, and animal cells (e.g., rodent cells, human cells, and non-human primate cells). Applicable target cells can be any type of cell, including stem cells, somatic cells, etc.

When the C2C9 nuclease, gRNA, system, composition, method, etc. described herein are applied to eukaryotic cells, the eukaryotic cells may be, for example, mammalian cells. In some embodiments, the cells are not human fetal cells or are not derived from human fetal cells. In some embodiments, the cells are not human embryonic cells or are not derived from human embryonic cells. In some embodiments, the present disclosure does not involve the destruction of human fetuses or human embryos. In some embodiments, the subject or individual is not a human fetus or embryo.

When the C2C9 nuclease or the polynucleotide encoding it, the gRNA or the polynucleotide encoding it, the recombinant expression vector, the system, the composition, and the kit described herein are applied to prokaryotic cells, the prokaryotic cells may be Escherichia coli. In some embodiments, they can turn off gene expression in a bacterial cell. In some embodiments, a nucleic acid encoding a suitable gRNA and/or a suitable C2C9 nuclease is introduced into a chromosome of a target cell, and translation and expression of the nucleic acid is carried out under the control of an inducible promoter, wherein the encoded gRNA and C2C9 nuclease form a complex that cleaves the target DNA site of interest. In some embodiments, the bacterial genome comprises a nucleic acid sequence encoding an appropriate C2C9 nuclease and/or an appropriate gRNA on a plasmid by genetic engineering, and under the control of an inducible promoter, the expression of any targeted genes is controlled by inducing the expression of the gRNA and the C2C9 nuclease. In some embodiments, the C2C9 nuclease has enzymatic activity that modifies the target DNA in ways other than introducing double-strand breaks. The enzymatic activity may be inherent to C2C9 itself, or obtained by, for example, fusing a heterologous polypeptide having an enzymatic activity to the C2C9 nuclease to form a chimeric C2C9 nuclease, and the enzymatic activity includes, but is not limited to, methyltransferase activity, deamidation activity, dismutase activity, alkylation activity, demethylase activity, DNA repair activity, transposase activity, recombinase activity, DNA damage activity, depurination activity, oxidation activity, pyrimidine dimer formation activity, and the like. In the present disclosure, a desired complementary nucleic acid sequence may be genetically engineered into the gene-targeting segment (e.g., a DNA-targeting segment) of the gRNA to control genetic modification at any position of the target gene. In the present disclosure, a desired complementary nucleic acid sequence may be genetically engineered into C2C9 to control genetic modification of target nucleic acid at any position in the target gene. In some embodiments, the application includes intentional and controlled damage to DNA at any desired position in the bacterial target DNA. In some embodiments, the application includes sequence-specific and controlled repair of DNA at any desired position in the bacterial target DNA.

The present disclosure also provides a method for targeting, editing, modifying, or manipulating a target gene (e.g., target DNA) in cells, in vivo or in isolated cells, or in a cell-free system, comprising: introducing the C2C9 nuclease or the polynucleotide encoding it, the gRNA or the polynucleotide encoding it, the recombinant expression vector, the system, the composition, the kit, etc. as described above into in vivo, isolated cells, or a cell-free system, and performing targeting, editing, modifying, or manipulating on the target gene.

In an embodiment, the method comprises the following:
(a) introducing the C2C9 nuclease or the nucleic acid encoding the C2C9 nuclease into in vivo, isolated cells, or cell-free systems; and
(b) introducing the gRNA (sgRNA) or a nucleic acid (e.g., DNA) suitable for the in situ production of such sgRNA; and
(c) contacting the cell or the target gene with the C2C9 nuclease or the nucleic acid encoding the C2C9 nuclease, and the gRNA (sgRNA) or the nucleic acid suitable for the in situ production of such sgRNA, to produce one or more cleavages, nicks, or edits in the target gene; wherein the C2C9 nuclease is directed to the target gene by processed or unprocessed form of gRNA.

In some embodiments, the target gene is a target DNA. In some embodiments, the target DNA may be in vitro naked DNA that is not bound to a DNA-associated protein. In some embodiments, the target DNA is chromosomal DNA in an in vitro cell. In some embodiments, the target gene is a target RNA. In some embodiments, the target DNA contacts with a targeting complex comprising the C2C9 nuclease and gRNA, the gRNA provides a target specificity to the targeting complex due to it's nucleotide sequence complementary to the target DNA, and the C2C9 nuclease provides the site-specific activity. In some embodiments, the targeting complex modifies the target DNA, resulting in, for example, DNA cleavage, DNA methylation, DNA damage, DNA repair, and the like. In some embodiments, the targeting complex modifies a target DNA-associated polypeptide (e.g., a histone, a DNA-binding protein, etc.), which results in, for example, methylation of the target DNA-associated polypeptide-histone, acetylation of histone, ubiquitination of histone, etc.

The method of present disclosure, when applied in vivo, the C2C9 nuclease or the polynucleotide encoding it, the gRNA or the polynucleotide encoding it, the recombinant expression vector, the system, the composition, and/or the donor polynucleotide are directly administered to the individual.

In the present disclosure, the C2C9 nuclease or a nucleic acid comprising the nucleotide sequence encoding a polypeptide of the C2C9 nuclease can be introduced into a cell by well-known methods. Similarly, the gRNA or a nucleic acid comprising the nucleotide sequence encoding gRNA can be introduced into the cell by well-known methods. Known methods include, DEAE-dextran-mediated transfection, liposome-mediated transfection, viral or bacteriophage infection, lipid transfection, transduction, conjugation, protoplast fusion, polyethyleneimine-mediated transfection, electroporation, calcium phosphate precipitation, gene gun, microinjection, nanoparticle-mediated nucleic acid delivery, and the like. Examples include delivery of plasmids by electroporation, calcium chloride transfection, microinjection, and lipid transfection. For viral vector delivery, the cells are brought into contact with viral particles comprising nucleic acids encoding gRNA and/or C2C9 nuclease, and/or nucleic acids of chimeric C2C9 nucleases, and/or donor polynucleotides.

In some embodiments, in the method of the present disclosure, the nuclease cleaves target DNA in a cell to produce a double-stranded break, which is then typically repaired by the cell in the following ways: non-homologous end joining (NHEJ) and homology-directed repair.

**In** non-homologous end-joining, the double-stranded break is repaired by directly interconnecting the broken ends. During the described process, a few base pairs can be inserted or deleted at the cleavage site. Thus, cleavage of DNA by C2C9 nuclease can be used to delete nucleic acid material from the target DNA sequence by cleaving the target DNA sequence and allowing the cell to repair the sequence in the absence of an exogenously provided donor polynucleotide. Thus, the method may be used to knock out a gene or knock genetic material into a selected locus in the target DNA.

In homology-directed repair, a donor polynucleotide having homology to the cleaved target DNA sequence is used as a template for repairing the cleaved target DNA sequence, resulting in the transfer of genetic information from the donor polynucleotide to the target DNA. Thus, new nucleic acid material can be inserted/replicated into the sites. In some embodiments, the target DNA is in contact with the donor polynucleotide. In some embodiments, the donor polynucleotide is introduced into the cell. Modifications to the target DNA caused by NHEJ and/or homology-directed repair result in, for example, gene correction, gene replacement, transgene insertion, nucleotide deletion, nucleotide insertion, gene disruption, gene mutation, sequence replacement, and the like.

In some embodiments, the cells comprising the target gene are in vitro. In some embodiments, the cell comprising the target gene is in vivo. Suitable nucleic acids comprising the nucleotide sequence encoding the gRNA and/or the C2C9 nuclease comprise an expression vector, wherein the expression vector comprising the nucleotide sequence encoding the gRNA and/or the C2C9 nuclease is a recombinant expression vector. In some embodiments, the C2C9 nuclease and one or more gRNAs are introduced into a cell via the same or different recombinant vectors to make the cell contact with the vector comprising the polynucleotide encoding the gRNA and/or the C2C9 nuclease, so as to allow the cell to absorb the vector.

The above method of the present disclosure can also be used to edit or modify DNA at multiple locations in a cell.

The above method of the present disclosure can also be used to regulate the transcription of target DNA.

In some embodiments, the method provided in the present disclosure comprises ligating a complete, or partially or completely defective C2C9 nuclease or a gRNA portion to a dimeric FOK1 nuclease, to guide endonuclease cleavage when being directed to one or more specific DNA target sites via one or more crRNA molecules. In some other embodiments, the method provided in the present disclosure involves introducing into a cell the C2C9 nuclease fused to the dimerized FOK1 nuclease along with two or more gRNAs that act as RNA or encoded DNA and are under the control of a promoter, wherein each gRNA comprises an array of spacer regions of repetitive sequences, wherein the spacer regions comprise nucleic acid sequences complementary to the target sequence in the DNA, and the repetitive sequences comprise stem-loop structures. In some embodiments, the method provided in the present disclosure comprises ligating a complete, or a partially or completely defective C2C9 nuclease, or a gRNA to a donor single-stranded or double-stranded DNA donor template, to facilitate homologous recombination of the exogenous DNA sequence by being directed to one or more specific DNA target sites via one or more gRNA molecules.

The present disclosure also provides a method for target gene-associated polypeptides, the method comprises contacting the target gene with the targeting complex, wherein the targeting complex comprises the C2C9 nuclease or the nucleic acid encoding the C2C9 nuclease, the gRNA or the nucleic acid suitable for the in situ production of such sgRNA as described above.

In some embodiments, the target gene-associated polypeptide is a histone, a DNA-binding protein, and the like. In some embodiments, the targeting complex modifies the target DNA-associated polypeptide, thereby resulting in, for example, methylation of the target DNA-associated polypeptide (such as histone), histone acetylation, histone ubiquitination, and the like.

In some embodiments, the target gene is a target DNA. In some embodiments, the target DNA is chromosomal DNA in an in vitro cell. In some embodiments, the target DNA is chromosomal DNA in an in vivo cell. In some embodiments, the target gene is a target RNA. In some embodiments, the target DNA contacts with the targeting complex comprising the C2C9 nuclease and gRNA. The gRNA and the C2C9 nuclease form the complex, wherein the gRNA proposes target specificity to the targeting complex by comprising the nucleotide sequence complementary to the target DNA, and the C2C9 nuclease provides site-specific activity. In some embodiments, the C2C9 nuclease based - CRISPR-C2C9 system involves modified polynucleotides, which modify DNA, RNA, and/or gRNA encoding the C2C9 nuclease introduced into the cell, so as to edit the locus of any one or more genomes.

The present disclosure also provides a cell, comprising those host cells that have been genetically modified with the above-described C2C9 nuclease or the polynucleotide encoding it, the gRNA or the polynucleotide encoding it, the recombinant expression vector, the system, or the composition.

Effective dosages of gRNA and/or C2C9 nuclease and/or recombinant expression vector and/or donor polynucleotide in the present disclosure are conventional to one skilled in the art. They can be determined by the different administration manners and the characteristics of the conditions being treated.

The C2C9 nuclease is a novel nuclease suitable for the CRISPR gene editing system developed by the applicant. It can accurately locate the target gene under the guidance of the corresponding guide RNA, perform genomic DNA cleavage, and realize double-stranded genomic DNA breaks. Variants of the C2C9 nuclease can have different or favorable characteristics. The C2C9 nuclease and its variant offer new opportunities for genome editing that did not exist before. The C2C9 nuclease and its variant can have different activities depending on the system in which they are used. Although the molecular weight of C2C9 is only half or even smaller than that of Cas9 and Cas12a, C2C9 has a genome cleavage efficiency close to or similar to that of Cas9 and Cas12a, which enables highly efficient genome editing in prokaryotic bacteria and eukaryotic cells, and thus has great application prospects in biotechnology, gene therapy, etc.

The C2C9 nuclease provided in the present disclosure has better performance compared to reported CRISPR-Cas endonucleases, such as higher activity in prokaryotic, eukaryotic, and/or in vitro environments. In some embodiments, C2C9 combines one or more of the following: small size, high editing activity, and a need for short PAM sequences. The small size of the RNA-directed nuclease refers to the nuclease with a length of no greater than about 800 amino acids.

In the present disclosure, the bacteria or prokaryotic bacteria may be Escherichia coli, Klebsiella pneumoniae, Bacteroides ovatus, Campylobacter jejuni, Staphylococcus saprophyticus, Enterococcus faecium, Bacteroides thetaiotaomicron, Bacteroides vulgatus, Bacteroides uniformis, Lactobacillus casei, Bacteroides fragilis, Acinetobacter baumannii, Fusobacterium nucleatum, Bacteroides dorei, Bacteroides acidifaciens, Lactobacillus rhamnosus, Bacteroides massiliensis, Bacteroides coprocola, Clostridium perfringens, and Bifidobacterium breve, etc.

In the present disclosure, the eukaryotic cells include, but are not limited to, mammalian cells, fungi, and the like. The fungi include yeasts, Aspergillus, such as Saccharomyces cerevisiae, Hansenula polymorpha, Picrosporum, Kluyveromyces fragilis, Kluyveromyces lactis, as well as Schizosaccharomyces pombe, Candida albicans, Candida dubliniensis, Candida glabrata, Candida guilliermondii, Candida kefyr, Candida krusei, Candida lusitaniae, Candida melinii, Candida oleophila, Candida parapsilosis, Candida tropicalis, and Candida utilis, Aspergillus fumigatus, Aspergillus flavus, Aspergillus niger, Aspergillus clavatus, Aspergillus glaucus, Aspergillus nidulans, Aspergillus oryzae, Aspergillus terreus, Aspergillus ustus, and Aspergillus versicolor.

In embodiments of the present disclosure, a novel genome editing method based on a small sized CRISPR/C2C9 nuclease is disclosed. The disclosure shows that through the direction and localization functions of the guide RNA, C2C9 is able to precisely cleave the genomic DNA and achieve genomic DNA double-strand breaks. Utilizing the host cell's own or exogenous repair mechanisms, the system can efficiently and precisely perform intracellular gene editing in living cells.

The implementations of the present disclosure are clearly and completely described below with reference to the embodiments. Obviously, the described embodiments are only intended to illustrate a part of the implementations of the present disclosure and should not be regarded as limiting the scope of the present disclosure. Where specific conditions are not indicated in the examples, conventional conditions or the manufacturer's recommended conditions are followed. Reagents or instruments without specified manufacturers are considered to be routine products that can be commercially purchased.

The sources of biomaterials used in embodiments are listed below:

Klebsiella pneumoniae NCTC9633 strain (purchased from ATCC, USA, catalog number: 13883); competent E. coli DH5α strain (purchased from CWBio Co., Ltd., catalog number: CW0808S). The LB liquid medium used in embodiments was purchased from Sangon Biotech (Shanghai) Co., Ltd., catalog number: A507002-0250; LB solid medium was purchased from Sangon Biotech (Shanghai) Co., Ltd.; Apramycin was purchased from BioVision, USA, catalog number: B1521-1G; and Kanamycin was purchased from Shanghai Aladdin Biochemical Technology Co., Ltd.

### Embodiment 1: Construction of plasmids p15a-AcC2C9Control and p15a-AcC2C9PAM for protospacer-adjacent motif (PAM) identification of AcC2C9

(1.1) The p15a-AcC2C9Control plasmid has a sequence shown as SEQ ID NO.124. It is constructed as follows:
The entire gene sequences of AcC2C9 nuclease and its corresponding guide RNA expression cassette (whose sequence is shown as SEQ ID NO. 125), as well as the p15a plasmid backbone (whose sequence is shown as SEQ ID NO.126) were synthesized at Sangon Biotech (Shanghai) Co., Ltd. The two DNA sequences were assembled into the p15a-AcC2C9Control plasmid using Gibson assembly technique. The plasmid was transformed into commercially competent E. coli DH5αcells and coated on LB medium plates with 75 µg/mL apramycin for screening. Single clones were picked for expansion culture, plasmid extraction was performed, and the plasmid sequence was verified by sequencing to obtain the p15a-AcC2C9Control plasmid.

(1.2) The p15a-AcC2C9PAM plasmid has a sequence shown as SEQ ID NO.127, which is constructed as follows:
the two primers below were synthesized at Sangon Biotech (Shanghai) Co., Ltd:
PAMprimerF: 5'-atcgTGTCCTCTTCCTCTTTAGCG-3' (SEQ ID NO.128)
PAMprimerR: 5'-ggccCGCTAAAGAGGAAGAGGACA-3' (SEQ ID NO. 129)

These two primers were annealed, and the specific reaction system was as follows: 5 µl 10×T4 DNA ligase Buffer (from NEB), 10 µL PAM primerF (10 µM), 10 µL PAM primerR (10 µM), 25 µL ddH₂O. The mixture was heated at 95°C for 5min and then slowly cooled to room temperature over 1~2 hours to allow the two single-stranded primers to form double-stranded DNA by base pairing. The resulting product was then diluted 20-fold with ddH₂O.

The obtained double-stranded DNA was inserted into the BsaI site of the p15a-AcC2C9Control plasmid by Golden Gate technology, and the specific reaction system was as follows: 1 µL 10×T4 DNA ligase Buffer, 1 µL of the 20-fold diluted phosphorylated double-stranded DNA, 20 fmol of p15a-AcC2C9Control plasmid, 0.5 µL of T4 DNA ligase (400 units/µL), 0.5 µL of BsaI-HF (20 units/µL), and an appropriate amount of ddH₂O was added to make the total volume of 10 µL. Buffer and enzymes used in this reaction were produced by NEB. The reaction was performed in a PCR instrument with the following cycle: 37°C for 2min; 16°C for 5min, for a total of 25 cycles; followed by 80°C for 15min.

10 µL of the reaction product was transformed into competent E. coli DH5α strains and coated on the LB solid culture plate containing 75 µg/mL apramycin. After the transformation mixture was absorbed by the solid culture plate, it was incubated upside down overnight at 37°C in an incubator. The resulting transformant colonies grown on the medium were transferred and preserved, and the plasmids were extracted and sent to Sangon Biotech (Shanghai) Co., Ltd for sequencing validation to obtain the p15a-AcC2C9PAM plasmid.

### Embodiment 2 Construction of substrate plasmid pUC19-6N-library for PAM identification

The pUC19-6N-library plasmid has a sequence shown as SEQ ID NO. 130. It is constructed as follows:
The commercial pUC19 plasmid, using as a template, the primer 6N-F with a 6N random sequence (whose sequence is shown as SEQ ID NO. 131), and the common primer 6N-R (whose sequence is SEQ ID NO. 132) were used for circular polymerase extension cloning (CPEC). The PCR products were subjected to digestion treatment with DpnI, and transformed into commercially competent Escherichia coli DH5αcells. The cells were coated on the LBA plate with carbenicillin, and more than 100,000 single colonies were collected by scraping with a spreader. The single colonies were mixed and the plasmids were extracted to obtain the PAM-identified substrate plasmid pUC19-6N-library.

### Embodiment 3 PAM Identification of AcC2C9

The p15a-AcC2C9Control plasmid constructed in Embodiment 1 was transformed into commercially competent E. coli DH5αcells and then coated on the LB solid plate containing 75 µg/mL apramycin. The next day, single colonies were picked and cultured in 100 mL of fresh LB liquid medium. When OD600=0.5, the bacteria were collected, then washed twice using pre-ice cold sterilized 10%v/v glycerol, and finally resuspended in 1 mL of sterilized 10%v/v glycerol solution to obtain competent DH5αcells with the p15a-AcC2C9Control plasmid.

The preparation of competent DH5αcells with the p 15a-AcC2C9P AM plasmid constructed in Embodiment 1 was the same way as the above preparation of competent DH5αcells with the p15a-AcC2C9Control plasmid.

100 ng of the pUC19-6N-library plasmid constructed in Embodiment 2 was electroporated into competent DH5αcells with the p15a-AcC2C9Control plasmid, and coated on the LB solid plate with 50 µg/mL carbenicillin and 75 µg/mL apramycin. More than 100,000 single colonies were collected by scraping with a spreader and then mixed. The plasmid was extracted to obtain the control library.

100 ng of pUC19-6N-library plasmid constructed in Embodiment 2 was electroporated into competent DH5αcells with p 15a-AcC2C9P AM plasmid to prepare a PAM depletion library for AcC2C9. The preparation was the same as that of the control library.

The control library and PAM depletion library, serving as templates, respectively, the primers PAM-1F (whose sequence is shown as SEQ ID NO. 133) and PAM-1R (whose sequence is shown as SEQ ID NO. 134) were used to perform the first round of PCR, and the product was detected by electrophoresis. Then 1 µL of the first-round PCR product, serving as a template, the primers PAM-2F (the sequence of which is shown as SEQ ID NO. 135) and PAM-2R (the sequence of which is shown as SEQ ID NO. 136) were used to perform the second round of PCR, and the product was detected by electrophoresis and purified using VAHTS DNA Clean Beads (Vazyme Biotech Co., Ltd). High-throughput sequencing was performed using the Illumina Hiseq2500 platform, 1 Gb of raw data was obtained, and 6N random sequence information was obtained. The sequence frequencies in the PAM depletion library were compared with the sequence frequencies in the control library to create a weblogo, thus obtaining the PAM information for the preference of AcC2C9.

Figure 1 shows the identification process and results of the PAM of AcC2C9. The results show that AcC2C9 can effectively recognize 5'-AAN type PAM sequences as well as a small number of 5'-GAN sequences (where N is a degenerate base, representing any one of the bases of A, T, C, and G). The horizontal coordinates -1, -2, -3, -4, -5, and -6 respectively represent the 1st, 2nd, 3rd, 4th, 5th, and 6th DNA bases located upstream of the 5' end of the target-DNA segment sequence on the NTS strand (non-targeting strand). The letters on these coordinates represent the preference of the AcC2C9 nuclease for the DNA sequence, and the larger the proportion of the letter, the stronger the preference of AcC2C9 for that base.

### Embodiment 4 PAM identification of CgC2C9, RdC2C9, and MiC2C9

The PAM identification procedures for CgC2C9, RdC2C9, and MliC2C9 were similar to that for AcC2C9.

The entire gene sequences of CgC2C9, RdC2C9, and MiC2C9 nucleases and their corresponding guide RNA expression cassettes (the sequences are shown as SEQ ID NO. 137-139) were synthesized at Sangon Biotech (Shanghai) Co., Ltd. These three nuclease DNA fragments were assembled separately using Gibson assembly technique with the p15a plasmid backbone (whose sequence is shown as SEQ ID NO. 126) to form the p15a-CgC2C9Control (the sequence is shown as SEQ ID NO. 140), the p15a-RdC2C9Control (the sequence is shown as SEQ ID NO. 141), and the p15a-MiC2C9Control (the sequence is shown as SEQ ID NO. 142). The p15a-CgC2C9Control, p15a-RdC2C9Control, and p15a-MiC2C9Control were respectively transformed into commercially competent E. coli DH5α cells, and then coated on the LB medium plates with 75 µg/mL apramycin for screening. Single colonies were picked for expansion culture, the plasmids were extracted and sequenced to verify plasmid sequences.

The following primers were synthesized by Sangon Biotech (Shanghai) Co., Ltd:
CgPAMprimerF: 5'-tcccTGTCCTCTTCCTCTTTAGCG-3' (SEQ ID NO. 143)
RdPAMprimerF: 5'-ttccTGTCCTCTTCCTCTTTAGCG-3' (SEQ ID NO. 144)
MiPAMprimerF: 5'-gcctTGTCCTCTTCCTCTTTAGCG-3' (SEQ ID NO. 145)

Primers corresponding to CgC2C9, RdC2C9, and MiC2C9 were annealed with PAMprimerR: 5'-ggccCGCTAAAGAGGAAGAGGACA-3' (SEQ ID NO. 129), respectively. The specific reaction system was as follows: 5 µl 10×T4 DNA ligase Buffer (from NEB), 10 µL primerF (10 µM), 10 µL primerR (10 µM), and 25 µL ddH₂O. The mixture was heated at 95oC for 5min, and then slowly cooled to room temperature over 1-2 hours to allow the two single-stranded primers to form double-stranded DNA by base pairing. The product obtained was then diluted 20-fold with ddH₂O. The double-stranded DNA obtained above was inserted into the BsaI sites of p15a-CgC2C9Control, p15a-RdC2C9Control, and p15a-MiC2C9Control plasmids, respectively, by using Golden Gate technology, and the specific reaction system was as follows: 1 µL of 10×T4 DNA ligase Buffer, 1 µL of 20-fold diluted phosphorylated double-stranded DNA, 20 fmol of plasmid, 0.5 µL T4 DNA ligase (400 units/µL), 0.5 µL BsaI-HF (20 units/µL), and an appropriate amount of ddH₂O was added to a total volume of 10 µL. Buffer and enzymes used in this reaction were produced by NEB. The reaction was performed in a PCR instrument with the following cycle: 37°C for 2min; 16°C for 5min, for a total of 25 cycles; followed by 80°C for 15min. 10 µL of reaction product was transformed into competent E. coli DH5α strains, and coated on the LB solid culture plate containing 75 µg/mL apramycin. The transformation mixture was absorbed by the solid culture plate and then incubated upside down overnight at 37°C in an incubator. The transformant colonies grown on the medium were transferred and preserved, and then, the plasmid was extracted and sent to Sangon Biotech (Shanghai) Co., Ltd. for sequencing validation, to finally obtain p15a-CgC2C9PAM (the sequence is shown as SEQ ID NO. 146), p15a-RdC2C9PAM (the sequence is shown as SEQ ID NO. 147), andp15a-MiC2C9PAM (the sequence is shown as SEQ ID NO. 148) plasmids.

PAM preference identification of CgC2C9, RdC2C9, and MiC2C9 was performed. The procedures were identical to the PAM identification procedures of AcC2C9 in Embodiment 3.

Figure 2 shows the PAM identification results of CgC2C9, RdC2C9, and MiC2C9. The results show that CgC2C9 (A) can effectively recognize 5'-AAS type PAM sequences(where S is a degenerate base, representing any one of C and G bases), and RdC2C9 (B) and MiC2C9 (C) can effectively recognize 5'-AAH type PAM sequences (where N is a degenerate base, representing any one of the bases of A, T, and C).

### Embodiment 5 Construction of p15a-AcC2C9 and pSGKP-AcC2C9 plasmids for genome editing of Klebsiella pneumoniae

The p15a-AcC2C9 plasmid has a sequence shown as SEQ ID NO. 149, which was constructed as follows:
the p15a-AcC2C9Control plasmid from Embodiment 1 was used as a template to amplify the AcC2C9 nuclease gene and the p15a plasmid backbone, respectively.
5' primer sequence AcC2C9F of amplification of the AcC2C9 nuclease gene:
   tcatctgtgcatatagctatactgatttcgtcagactcaca (SEQ ID NO. 150)
   3' primer sequence ACC2C9R of amplification of the AcC2C9 nuclease gene:
   cgccaaccagccaCCTTATTGACCTGACCGCGCCTATG (SEQ ID NO. 151)
   5' primer sequence p15aF of amplification of the p15a plasmid backbone:
   CAGGTCAATAAGGtggctggttggcgtactgtt (SEQ ID NO. 152)
   3' primer sequence plSaR of amplification of the p15a plasmid backbone:
   atcagtatagctatatgcacagatgaaaacggtgtaaaaaaga (SEQ ID NO. 153)

The AcC2C9 gene fragment, as well as the p15a plasmid backbone were amplified using Phanta Max Master Mix reagents from Vazyme Biotech Co., Ltd, and the reaction system included 25 µL 2xPhanta Max Master Mix, 1.5 µL 5'Primer (10 µM), 1.5 µL 3 'Primer (10 µM), 0.5 µl template DNA (100 ng/µL), 1.5 µL DMSO, and 20 µL ddH₂O. After the PCR reaction system was prepared, polymerase chain reaction (PCR) was carried out with the following cycle: 98°C for 2min; then 98°C for 20s, 55°C for 20s, and 72°C for 3min, for a total of 30 cycles; followed by 72°C for 5min. PCR products were recovered using the SanPrep column PCR product purification kit produced by Sangon Biotech (Shanghai) Co., Ltd. Specific steps for PCR product purification were carried out in accordance with the kit's instruction manual.

The resulting AcC2C9 gene fragment and p15a plasmid backbone were assembled into a plasmid by Gibson Assembly technique. The specific reaction system included: 5 µL NEBuilderHiFi DNA Assembly Master Mix (from NEB), 20 fmol of AcC2C9 gene fragment, 20 fmol of p15a plasmid backbone, and an appropriate amount of ddH₂O was added to a total volume of 10 µL. The reaction was carried out at 50°C for 1 hour. 10 µL of the reaction product was transformed into competent E. coli DH5α strains, and coated on the LB solid culture plate containing 75 µg/mL apramycin. The transformation mixture was absorbed by the solid culture plate and then incubated upside down at 37°C overnight in an incubator. The transformant colonies grown on the culture plate were transferred and preserved, and the plasmids were extracted for subsequent experiments using the SanPrep Column Plasmid DNA Mini-Prep Kit from Sangon Biotech (Shanghai) Co., Ltd. The specific steps for plasmid extraction were carried out in accordance with the instruction manual of the kit. The plasmid was then sent to Sangon Biotech (Shanghai) Co., Ltd. for sequencing validation to obtain the p15a-AcC2C9 plasmid.

The pSGKP-AcC2C9 plasmid has a sequence shown as SEQ ID NO.154, which was constructed as follows:

The entire gene sequence of the guide RNA expression cassette (the sequence is shown as SEQ ID NO.155) corresponding to the AcC2C9 nuclease and the pSGKP plasmid backbone (the sequence is shown as SEQ ID NO.156) were synthesized at Sangon Biotech (Shanghai) Co., Ltd. The two DNA fragments were assembled into the pSGKP-AcC2C9 plasmid using Gibson assembly technique. The plasmid was transformed into commercially competent E. coli DH5αcells, coated on the LB medium plate with 50 µg/mL kanamycin for screening. Single colonies were picked for expansion culture, the plasmids were extracted and sequenced to verify the plasmid sequences.

### Embodiment 6 Construction of pSGKP-AcC2C9-dha plasmid targeting dha gene of Klebsiella pneumoniae genome

The pSGKP-AcC2C9-dha plasmid has a sequence shown as SEQ ID NO.157, which was constructed as follows:
a DNA fragment consisting of 20 bases following a particular AAG (the PAM sequence recognized by AcC2C9 was selected as the AAG in this Embodiment) sequence in the Klebsiella pneumoniae target gene was first selected (these 20 bases are referred to as the spacer, and the AAN is not included), and then the spacer sequence was inserted into the p15a- AcC2C9Control plasmid constructed in Embodiment 1. This step is characterized by the need to add atcg to the 5' end of the single-stranded DNA sequence to insert the spacer fragment into the BsaI site of the p15a-AcC2C9Control plasmid. Additionally, the reverse complement sequence of the spacer needed to be synthesized with ggcc added to its 5' end. For example, if the DNA sequence (SEQ ID NO. 158) of the selected spacer of the dha gene in this embodiment is: 5'-TGTTTGTTACCAACTGCCTG-3', the specific sequences of the two primers are designed as follows:
PAM primerF (dha-spF): 5'-atcgTGTTTGTTACCAACTGCCTG -3' (SEQ ID NO. 159)
PAM primerR (dha-spR): 5'-ggccCAGGCAGTTGGTAACAAACA -3' (SEQ ID NO. 160).

The two primers mentioned above were annealed, and the specific reaction system was as follows: 5 µl 10×T4 DNA ligase Buffer (from NEB), 10 µL PAM primerF (10 µM), 10 µL PAM primerR (10 µM), and 25 µL ddH₂O. The mixture was heated to 95°C for 5min and then slowly cooled to room temperature over 1-2 hours to allow the two single-stranded primers to form double-stranded DNA by base pairing. The product obtained was then diluted 20-fold with ddH₂O.

The double-stranded DNA obtained above was inserted into the BsaI site of the pSGKP-AcC2C9 plasmid constructed in Embodiment 4 by Golden Gate technology, and the specific reaction system was as follows: 1 µL of 10×T4 DNA ligase Buffer, 1 µL of the 20-fold diluted phosphorylated double-stranded DNA described above, 20 fmol of pSGKP-AcC2C9 plasmid, 0.5 µL of T4 DNA ligase (400 units/µL), 0.5 µL BsaI-HF (20 units/µL), and finally, an appropriate amount of ddH₂O was added to a total volume of 10 µL. Buffer and enzymes used in this reaction were produced by NEB. The reaction was performed in a PCR instrument with the following cycle: 37°C for 2min; 16°C for 5min, for a total of 25 cycles; followed by 80°C for 15min.

10 µL of reaction product was transformed into competent E. coli DH5α strains, and coated on the LB solid culture plate containing 75 µg/mL apramycin. The transformation mixture was absorbed by the solid culture plate and then incubated upside down at 37°C overnight in an incubator. The resulting transformant colonies grown on the medium was transferred and preserved, and the plasmid was extracted and sent to Sangon Biotech (Shanghai) Co., Ltd. for sequencing validation to obtain the pSGKP-AcC2C9-dha plasmid.

### Embodiment 7 Preparation of electrocompetent Klebsiella pneumoniae containing p15a-AcC2C9 plasmid

The Klebsiella pneumoniae strain NCTC9633 (purchased from ATCC, USA, catalog no. 13883) was streaked on the LB solid culture plate and incubated upside down at 37°C overnight in an incubator. One single colony grown on the culture plate was picked and inoculated into 3 mL of LB liquid medium, and then shaken at 250 rpm in a 37°C shaker overnight. The next day, 1 mL of the bacterial liquid was inoculated into 100 mL of fresh LB liquid medium, and then shaken in a 37°C shaker. When the OD600 of the bacterial liquid reached 0.3, the bacterial liquid was placed on ice and cooled for ten minutes. The cells were collected by centrifugation at 8000 rpm for 5 min at 4°C, and the supernatant was discarded. The precipitate at the bottom was resuspended in 20 mL of 10% (v/v) glycerol (sterilized and pre-cooled on ice). The cells were centrifuged again at the same speed, and the supernatant was discarded. The precipitate at the bottom was resuspended again with 20 mL of 10% (v/v) glycerol. The cells were centrifuged again at the same speed, and the supernatant was discarded. The precipitate at the bottom was resuspended with 1 mL of 10% (v/v) glycerol to obtain electrocompetent cells. The electrocompetent cells were aliquoted into EP tubes with 50 µL of cell liquid per tube. The aliquoted cell liquid was rapidly frozen in liquid nitrogen and then stored in a -80°C refrigerator. It is important to note that resuspension should be done gently, using a pipette to gently pipette up and down to resuspend the cells.

One tube of freshly prepared electrocompetent Klebsiella pneumoniae was placed on ice for 5 to 10 minutes, and 1 µg of the p15a-AcC2C9 plasmid prepared in Embodiment 4 was added. After mixing, the mixture was transferred to a 1 mm electroporation cuvette (Bio-Rad) and subjected to electroporation at room temperature in a GenePulserXcell electroporation system (Bio-Rad). The electroporation parameters were: 1800 V, 200 S2, 25 µF. After electroporation, 1 mL of LB liquid medium was immediately added, mixed thoroughly, and transferred to a clean EP tube, and shaken in a 37°C shaker for 1 to 2 hours. 100 µL of the bacterial liquid was spread on the LB solid culture plate containing 75 µg/mL apramycin. After the bacterial liquid was absorbed by the solid culture plate, it was incubated upside down overnight in a 37°C incubator. One single colony was picked and cultured in 3 mL of LB liquid medium containing 75 µg/mL apramycin at 250 rpm in a shaker at 37°C overnight. The next day, 1 mL of bacterial liquid was inoculated into 100 mL of fresh LB medium containing 75 µg/mL apramycin, and shaken at 37°C in a shaker. When the OD600 of the bacterial liquid reached 0.2, 1 mL of 20% w/v arabinose was added to the bacterial liquid, and the bacterial liquid was shaken for another 2h. The liquid was placed on ice for ten minutes, and centrifuged at 8000 rpm for 5 min at 4°C to harvest the bacterial cells. The supernatant was discarded, and the precipitate at the bottom was resuspended and washed with 20 mL of 10% v/v glycerol (sterilized and pre-cooled on ice) twice. After a final centrifugation at the same speed, the supernatant was discarded, and the precipitate at the bottom was resuspended with 1 mL of 10% v/v glycerol. The obtained electrocompetent cell liquid was aliquoted into EP tubes with 50 µL of cell liquid per tube. The aliquoted cell liquid was rapidly frozen in liquid nitrogen and then stored in a -80°C refrigerator. It is important to note that resuspension should be done gently, using a pipette to gently pipette up and down to resuspend the cells.

### Embodiment 8 Highly efficient gene deletion in live Klebsiella pneumoniae using the AcC2C9 system

**A** tube of electropocompetent Klebsiella pneumoniae cells containing the p15a-AcC2C9 plasmid prepared in Embodiment 6 was placed on ice for 5∼10 minutes. After thawing, 200 ng of the pSGKP-AcC2C9-dha plasmid prepared in Embodiment 5 and the dha gene homologous recombination repair template (the sequence is shown as SEQ ID NO.161) at a final concentration of 5 µM were added and gently mixed. The uniform bacterial-plasmid mixture was transferred into a pre-cooled 1 mm electroporation cuvette (Bio-Rad) using a pipette and left on ice for 5 min. Condensation on the outside of the electroporation cuvette was wiped off, electroporation was performed using a GenePulserXcell electroporation system (Bio-Rad). The electroporation parameters were: 1800 V, 200 S2, 25 µF. After electroporation, 1 mL of LB culture solution was immediately added to wash out the cells. The cells then were transferred into sterile EP tubes and incubated for 1h in a 37°C shaker at 250 rpm/min. The bacterial liquid was spread on the LB solid medium containing 75 µg/mL apramycin and 50 µg/mL kanamycin, and after the bacterial liquid was absorbed, it was incubated upside down overnight in a 37°C incubator.

Colony PCR was performed on the resulting colonies to verify the editing result.

The 5' primer for colony PCR validation of the dha gene:
5 '- TCATCATTGGCATCCTGAAA-3' (SEQ ID NO. 162).

The 3' primer for colony PCR validation of the dha gene:
5 '- TGGTCAGCCTGCTGATTAAA-3' (SEQ ID NO. 163).

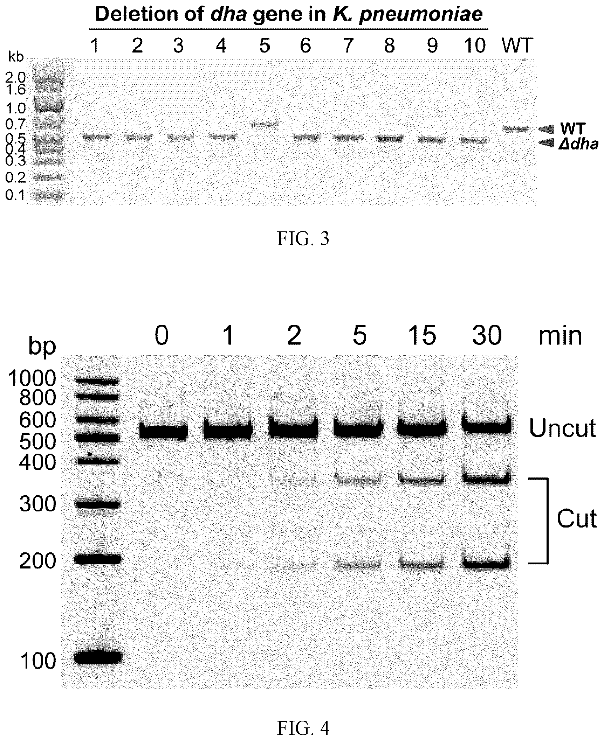

Figure 3 shows the PCR results of gene knockout in live Klebsiella pneumoniae cells using CRISPR-AcC2C9 system. The results show that the selected dha gene can be precisely knocked out by the CRISPR-AcC2C9 system.

### Embodiment 9 Construction of AcC2C9 protein heterologous expression plasmid pET28a-sumo-AcC2C9

The p15a-AcC2C9Control plasmid from Embodiment 1 was used as a template to amplify the AcC2C9 nuclease gene sequence for the construction of the pET28a-sumo-AcC2C9 plasmid that expresses AcC2C9 (the sequence of which is shown as SEQ ID NO. 164).

5' primer sequence pET-AcC2C9F of amplification of the AcC2C9 gene (5' Primer):
5'-tCTTGAAGTCCTCTTTCAGGGACCCATGGTGCAGACGGAGATCCTG-3' (SEQ ID NO. 165).

3' primer sequence pET-AcC2C9R of amplification of the AcC2C9 gene (3' Primer):
5'-gtcgacggagctcgaattcttaagcTTATGGAGGCGTTGCCGTTTCG-3' (SEQ ID NO. 166).

The AcC2C9 gene fragment was amplified using the Phanta Max Master Mix reagent from Vazyme Biotech Co., Ltd, and the reaction system was: 25 µL 2xPhanta Max Master Mix, 1.5 µL 5'Primer (10 µM), 1.5 µL 3'Primer (10 µM), 0.5 µl p15a-AcC2C9 plasmid (100 ng/µL) prepared in Embodiment 4, 1.5 µL DMSO, 20 µL ddH₂O. After the PCR reaction system was prepared, polymerase chain reaction (PCR) was carried out with the following cycle: 98°C for 2min; then 98°C for 20s, 55°C for 20s, and 72°C for 1min, for a total of 30 cycles; followed by 72°C for 5min. PCR products were recovered using the SanPrep column PCR product purification kit produced by Sangon Biotech (Shanghai) Co., Ltd. Specific steps for PCR product purification were carried out in accordance with the kit's instruction manual.

The entire gene sequence of the pET28a-sumo plasmid backbone was synthesized at Sangon Biotech (Shanghai) Co., Ltd. (the sequence is shown as SEQ ID NO. 167). The AcC2C9 gene fragment and the pET28a-sumo plasmid backbone were assembled into the pET28a-sumo-ACC2C9 plasmid using Gibson assembly technique. The plasmid was transformed into competent Escherichia coli DH5αcells, coated on the LB medium plate containing 50µg/mL kanamycin for selection. The single colonies were picked for expansion culture, the plasmid was extracted and sequenced to verify the plasmid sequence to obtain the pET28a-sumo-AcC2C9 plasmid.

### Embodiment 10 AcC2C9 efficiently achieves in vitro double-stranded DNA cleavage

Cleavage substrate preparation: the genome of Klebsiella pneumoniae was used as a template, and the cleavage substrate was amplified using 5' FAM labeled primer. The 5' primer sequence for amplifying the cleavage substrate was 5'-CCGCATATATCGCAAAACAA-3' (SEQ ID NO. 168), and the 3' primer sequence for amplifying the cleavage substrate was 5'-CTGGCTGTGAACAAAGTGGA-3' (SEQ ID NO. 169). PCR products were recovered using the SanPrep column PCR product purification kit produced by Sangon Biotech (Shanghai) Co., Ltd.

Preparation of AcC2C9 nuclease: the pET28a-sumo-AcC2C9 plasmid constructed in Embodiment 8 was transformed into the E. coli expression strain BL21 (DE3). On the next day, the transformants were transferred into 1 L of LB medium and incubated at 37°C with shaking. When the OD600 reached 0.6, 0.5 mL of 1 M IPTG was added to the culture, and the temperature was lowered to 16°C for overnight incubation. The overnight cultures were collected, and the cells were lysed by ultrasonic. The HisTrap Ni-NTA (GE Healthcare) affinity column was used for purification, followed by further purification using HiLoad 16/600 Superdex 200pg molecular sieve (GE Healthcare). The purified protein was concentrated using ultrafiltration tubes and stored in a buffer of 500 mM NaCl, 10 mMTris-HCl, pH=7.5, 1 mM DTT.

Preparation of the complete guide RNA (the sequence is shown as SEQ ID NO. 170) targeting the cleavage substrate: the preparation of the complete guide RNA was achieved by in vitro transcription. The transcription template DNA was synthesized by Genewiz Biotechnology Co., Ltd., and its sequence was shown as SEQ ID NO. 171. The complete guide RNA was transcribed using the HiScribe T7 High Yield RNA Synthesis Kit (from NEB) with the above templates. Specific steps were followed in accordance with the instruction manual of the kit. The prepared complete guide RNA was purified by phenol-chloroform extraction and ethanol precipitation.

In vitro cleavage experiment was performed under the condition of 150 mM NaCl, 10 mM MgCl₂, 10 mM Tris-HCl, pH=7.5, 1 mM DTT. The total reaction volume was 20 µL, which included 30 nM cleavage substrate, 2 µM AcC2C9, and 2 µM complete guide RNA. The reaction time was 0 min, 1 min, 2 min, 5 min, 15 min, and 30 min, respectively, and the reaction temperature was 37°C. The reaction was terminated by adding 50 mM EDTA and 2 mg/mL ProteinK. The reaction products were separated using a 6% w/v TBE acrylamide gel and imaged using a GELDOC gel imaging system (Bio-Rad) with 488nm blue light excitation.

Figure 4 shows the results of cleaving the substrate DNA using AcC2C9 nuclease. The results show that the substrate DNA was precisely cleaved into two DNA fragments by AcC2C9.

### Embodiment 11 Determination of optimal reaction condition for AcC2C9

In order to determine the optimal reaction conditions for AcC2C9, the present disclosure conducts in vitro cleavage assays to detect the activity of AcC2C9 under different reaction conditions.

The cleavage substrate was prepared as the description in Embodiment 10.

The AcC2C9 nuclease was prepared as the description in Embodiment 10.

The complete guide RNA targeting the cleavage substrate was prepared as the description in Embodiment 10.

### (1) Effect of temperature on the activity of AcC2C9

The in vitro cleavage experiments were performed under the condition of 100 mM NaCl, 10 mM MgCl₂, 10 mM Tris-HCl, pH=7.5, 1 mM DTT. The total reaction volume was 20 µL, which included 30 nM cleavage substrate, 2 µM AcC2C9, and 2 µM complete guide RNA. The reaction time was 0 min, 5 min, 10 min, 20 min, 40 min, and 60 min, respectively, and the reaction temperatures were 30 °C, 34 °C, 37 °C, 40 °C, 43 °C, 46 °C, and 50 °C, respectively. The reaction was terminated by adding 50 mM EDTA and 2 mg/mL ProteinK. The reaction products were separated using a 6% w/v TBE acrylamide gel and imaged using a GELDOC gel imaging system (Bio-Rad) with 488nm blue light excitation. The cleavage efficiency of AcC2C9 was determined by the ratio of the amount of cleaved bands to the amount of total DNA substrate.

### (2) Effect of NaCl concentration on the activity of AcC2C9

The reaction buffer for the in vitro cleavage experiment included 10 mM MgCl₂, 10 mM Tris-HCl, pH 7.5, and 1 mM DTT. 50 mM, 100 mM, 200 mM, 300 mM, and 500 mM NaCl were then added to the buffer, respectively. The total reaction volume was 20 µL, which included 30 nM cleavage substrate, 2 µM AcC2C9, and 2 µM complete guide RNA. The reaction time was 0, 5, 10, 20, 40, and 60 minutes, and the reaction temperature was 37°C. The reaction was terminated by adding 50 mM EDTA and 2 mg/mL ProteinK. The reaction products were separated using a 6% w/v TBE acrylamide gel and imaged using a GELDOC gel imaging system (Bio-Rad) with 488nm blue light excitation. The cleavage efficiency of AcC2C9 was determined by the ratio of the amount of cleaved bands to the amount of total DNA substrate.

### (3) Effect of MgCl₂ concentration on the activity of AcC2C9

The reaction buffer for the in vitro cleavage experiment included 100 mM NaCl, 10 mM Tris-HCl, pH=7.5, and 1 mM DTT. 0 mM, 1 mM, 2 mM, 5 mM, 10 mM, and 20 mM MgCl₂ were then added to the buffer. The total reaction volume was 20 µL, which included 30 nM cleavage substrate, 2 µM AcC2C9, and 2 µM complete guide RNA. The reaction time was 0, 5, 10, 20, 40, and 60 minutes, and the reaction temperature was 37°C. The reaction was terminated by adding 50 mM EDTA and 2 mg/mL ProteinK. The reaction products were separated using a 6% w/v TBE acrylamide gel and imaged using a GELDOC gel imaging system (Bio-Rad) with 488nm blue light excitation. The cleavage efficiency of AcC2C9 was determined by the ratio of the amount of cleaved bands to the amount of total DNA substrate.

### (4) Effect of different divalent ions on the activity of AcC2C9

The reaction buffer for the in vitro cleavage experiment included 100 mM NaCl, 10 mM Tris-HCl, pH=7.5, and 1 mM DTT. 10 mM of MgCl₂, CdCl₂, CoCl₂, MnCl₂, and NiCl₂ were then added to the buffer, respectively. The total reaction volume was 20 µL, which included 30 nM cleavage substrate, 2 µM AcC2C9, and 2 µM complete guide RNA. The reaction time was 0, 5, 10, 20, 40, and 60 minutes, and the reaction temperature was 37°C. The reaction was terminated by adding 50 mM EDTA and 2 mg/mL ProteinK. The reaction products were separated using a 6% w/v TBE acrylamide gel and imaged using a GELDOC gel imaging system (Bio-Rad) with 488nm blue light excitation. The cleavage efficiency of AcC2C9 was determined by the ratio of the amount of cleaved bands to the amount of total DNA substrate.

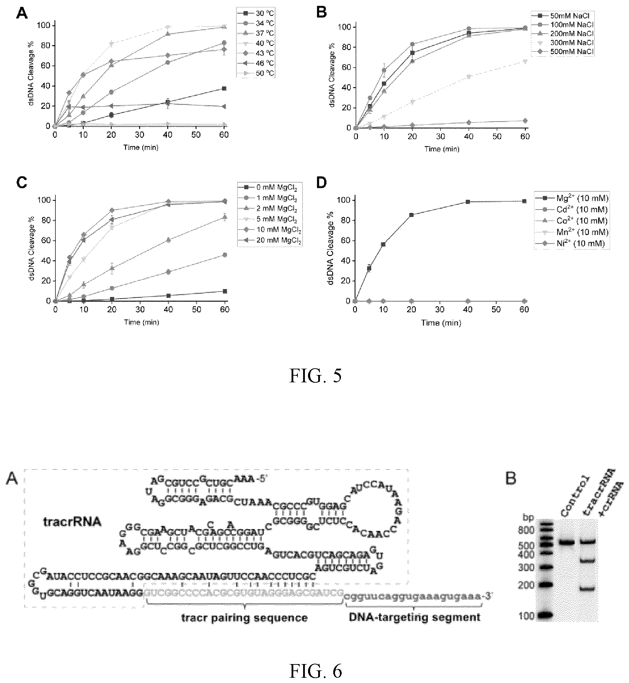

Figure 5 shows the reaction activity of AcC2C9 nuclease at different temperatures (A), different NaCl concentrations (B), different MgCl₂ concentrations (C), and different divalent ions (D). The results show that AcC2C9 has the highest activity at 40°C, 100 mM NaCl, and 10 mM MgCl₂. In the case of a 40-min reaction, AcC2C9 achieves a cleavage efficiency of 98.8% at 40 °C, 98.7% at 100 mM NaCl, and 98.9% at 10 mM MgCl₂.

### Embodiment 12 Detection of the activity after cleaving the complete guide RNA into tracrRNA and crRNA.

The complete guide RNA that targets the cleavage substrate includes a tracrRNA as well as a crRNA (a RNA sequence formed by linking the DNA-targeting segment hybridizing to the target sequence to the tracr pairing sequence). In order to verify whether the complete guide RNA still retained biologically active after being cleaved into tracrRNA and crRNA, an in vitro double-stranded DNA cleavage experiment was conducted in the present disclosure.

The cleavage substrate was prepared as the description in Embodiment 10.

The AcC2C9 nuclease was prepared as the description in Embodiment 10.

Preparation of tracrRNA (SEQ ID NO. 172): the preparation of tracrRNA was achieved by in vitro transcription. The transcription template DNA was synthesized by Genewiz Biotechnology Co.,Ltd., and its sequence is shown as SEQ ID NO. 173. The tracrRNA was transcribed using the HiScribe T7 High Yield RNA Synthesis Kit (from NEB) with the above template. Specific steps were followed in accordance with the instruction manual of the kit. The prepared tracrRNA was purified by phenol-chloroform extraction and ethanol precipitation.

Preparation of crRNA (SEQ ID NO. 174): the preparation of crRNA was achieved by in vitro transcription. The transcription template DNA was synthesized by Genewiz Biotechnology Co., Ltd., and its sequence is shown as SEQ ID NO. 175. The transcription and purification steps for crRNA were the same as those of tracrRNA.

In vitro cleavage experiment was performed under the condition of 150 mM NaCl, 10 mM MgCl₂, 10 mMTris-HCl, pH=7.5, 1 mM DTT. The total reaction volume was 20 µL, which included 30 nM cleavage substrate, 2 µM AcC2C9, 2 µM tracrRNA, and 2 µM crRNA. The reaction time was 30 minutes and the reaction temperature was 37°C. The reaction was terminated by adding 50 mM EDTA and 2 mg/mL ProteinK. The reaction products were separated using a 6% w/v TBE acrylamide gel and imaged using a GELDOC gel imaging system (Bio-Rad) with 488nm blue light excitation. It can be seen that under the conditions with crRNA and tracrRNA, the substrate DNA can similarly and precisely be cleaved into two DNA fragments, indicating that the complete guide RNA retained biologically active after being cleaved into tracrRNA and crRNA.

Figure 6 (A) shows a schematic sequence of the complete guide RNA of AcC2C9, and figure 6 (B) shows the results of cleavage of substrate DNA by AcC2C9 nuclease under the conditions with tracrRNA and crRNA.

### Embodiment 13 Identification of cleavage sites in double-stranded DNA by AcC2C9

The complete guide RNA targeting short fluorescently labeled synthetic DNA was prepared as the description in Embodiment 9.

The AcC2C9 nuclease was prepared as the description in Embodiment 9.

Preparation of cleavage substrate: the cleavage substrates were four 58 bp fluoresc ently labeled synthetic DNA sequences. Two complementary base-paring primers were sy nthesized by Sangon Biotech (Shanghai) Co., Ltd. with sequences of 5'-GGCTGTGAGAA AGCGGTTCAGGTGAAAGTGAAAACACTGCCCGACGCCCAGTTCGAAG-3' (SEQ ID NO. 176) and 5'- CTTCGAACTGGGCGTCGGGCAGTGTTTTCACTTTCACCTGAA CCGCTTTCTCACAGCC-3' (SEQ ID NO. 177). The FAM fluorescent groups were label ed at either the 5' or 3' end of each primer.

The in vitro cleavage experiment was performed under the conditions of 150 mM NaCl, 10 mM MgCl₂, 10 mM Tris-HCl, pH=7.5, 1 mM DTT. The total reaction volume was 10 µL, which included 20 nM cleavage substrate, 2 µM AcC2C9, and 2 µM complete guide RNA. The reaction temperature was 37°C. The reaction time was 30 minutes. The reaction was terminated by the addition of 10 µL of 2×formamide loading buffer (Sangon Biotech (Shanghai) Co., Ltd.). The reaction products were separated by 20% TBE-Urea-PAGE and imaged using 488 nm blue light excitation.

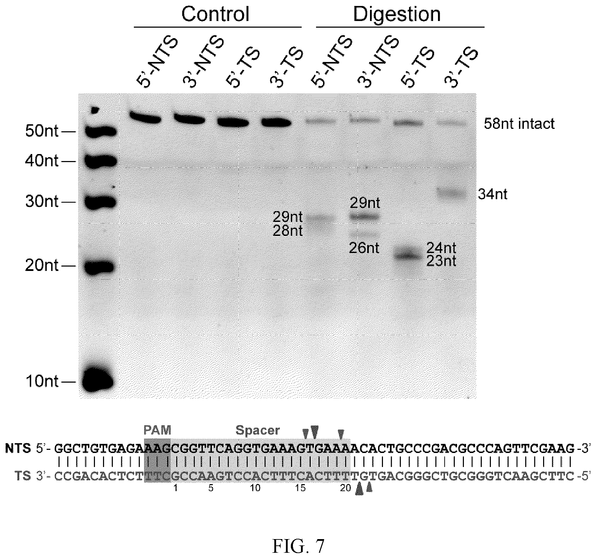

Figure 7 shows the results of AcC2C9 nuclease cleavage of four 58 bp fluorescently labeled DNA substrates. The results show that the major cleavage site of AcC2C9 in the targeting strand was located 21-22 nt downstream of the 3' end of the PAM site, whereas the major cleavage site in the non-targeting strand was located 15-16 nt downstream of the 3' end of the PAM site.

### Embodiment 14 Construction of mammalian cell gene editing plasmid pAcC2C9Hs for AcC2C9

The AcC2C9 in this embodiment was an AcC2C9 encoding gene optimized for human codons.

The sequence of pAcC2C9Hs plasmid is shown as SEQ ID NO. 178, which was constructed as follows:
The human-derived transient expression plasmid backbone (the sequence is shown as SEQ ID NO. 179), the puromycin resistance gene expression cassette sequence (the sequence is shown as SEQ ID NO. 180), the AcC2C9 encoding gene expression cassette optimized for human codons (the sequence is shown as SEQ ID NO. 181), and the corresponding guide RNA (the sequence is shown as SEQ ID NO. 123) expression cassette for AcC2C9 in human cells (the sequence is shown as SEQ ID NO. 182) were synthesized by Sangon Biotech (Shanghai) Co., Ltd. The four fragments were assembled into the pAcC2C9Hs plasmid by Gibson assembly technology. The plasmid was transformed into competent Escherichia coli DH5αcells, and coated on the LBA plate containing carbenicillin. The single colonies were picked for expansion culture, the plasmid was extracted and sequenced to obtain the pAcC2C9Hs plasmid.

### Embodiment 15 Efficient gene editing in living human cells using AcC2C9

Efficient gene editing in human live cells can be achieved using the AcC2C9 nuclease. Human embryonic kidney cell HEK293 was used as a model to demonstrate the gene editing effect.

Insertion of target spacer sequence into the pAcC2C9Hs plasmid: a total of five genes in the human genome, VEGFA, EMX1, HEXA, DNMT1, and FANCF, were selected as target sequences in this embodiment, and one 20 bp target sequence DNA was selected for each of these target sequences. The oligonucleotide sequences Guide1-F, Guide1-R, Guide2-F, Guide2-R, Guide3-F, Guide3-R, Guide4-F, Guide4-R, Guide5-F, and Guide5-R were synthesized (the sequences are shown as SEQ ID Nos. 183-192). After annealing the Guide1-F and Guide1-R, Guide2-F and Guide2-R, Guide3-F and Guide3-R, Guide4-F and Guide4-R, and Guide5-F and Guide5-R, respectively, the five target sequence DNAs were inserted into the pAcC2C9Hs plasmid by Golden gate assembly technique to construct the pAcC2C9Hs-guide1, pAcC2C9Hs-guide2, pAcC2C9Hs-guide3, pAcC2C9Hs-guide4, and pAcC2C9Hs-guide5 plasmids (sequences are shown as SEQ ID NO.193-197).

Gene editing of human cells mediated by transient expression plasmid: cryopreserved HEK293 cells were thawed and activated. Two days later, HEK293 cells were passaged into a 24-well plate with approximately 1.5 × 10⁵ cells per well. After 16-18 h, each plasmid (pAcC2C9Hs-guide1, pAcC2C9Hs-guide2, pAcC2C9Hs-guide3, pAcC2C9Hs-guide4, and pAcC2C9Hs-guide5) was transfected into the cells using 0.75 µL of Lipofectamine 3000 (Invitrogen). The cells were then incubated for 72 hours. Adherent cells were digested and genomic DNA was extracted. Gene-specific amplification primers (Guide1-IndelF and Guide1-IndelR, Guide2-IndelF and Guide2-IndelR, Guide3-IndelF and Guide3-IndelR, Guide4-IndelF and Guide4-IndelR, GuideS-IndelF and Guide5-IndelR, with sequences shown as SEQ ID NO. 198-207) were used to PCR to amplify the gene fragments. Gel recovery was performed to recover the PCR products. PCR products were then annealed using NEBuffer2 (from NEB). T7 endonuclease 1 (from NEB) was then added for enzymatic digestion at 37°C for 15 min. The reaction was terminated by the addition of 50 mM EDTA, the reaction products were separated by 6% TBE-PAGE and stained for imaging by 4S Red dye (Sangon Biotech (Shanghai) Co., Ltd.)

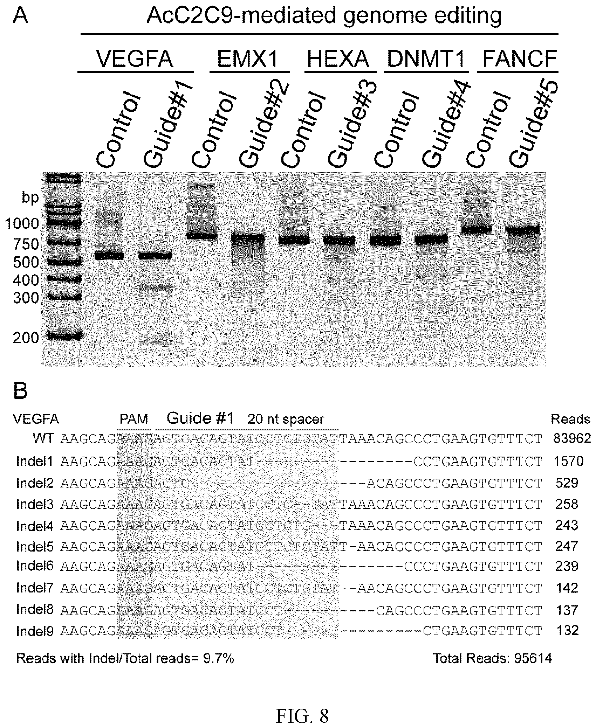

Figure 8 shows the results of gene editing in human cells mediated by the transient expression plasmid of AcC2C9. Figure 8(A) shows TBE-PAGE gel plot of the Indel experiment. The AcC2C9 nuclease successfully achieved efficient gene editing of VEGFA, HEXA, and DNMT1 genes in the five different gene sites selected. Figure 8(B) shows statistical results of high-throughput sequencing after VEGFA gene editing in human cells mediated by transient expression plasmid of AcC2C9. It can be observed that the AcC2C9 nuclease can precisely introduce insertions or deletions to the target sequence.

### Embodiment 16 Detection of editing efficiency of AcC2C9 at multiple sites in human live cells

To validate the generalizability of AcC2C9 at different gene loci, 47 additional targeting loci were selected at the VEGFA, AAVS1, PDCD1, HEXA, EMX1, TP53, DNMT1, and FANCE gene target sequences in the human genome. Guide6-F-Guide52-F(sequences are shown as SEQ ID NO.208-254) and Guide6-R-Guide52-R (sequences are shown as SEQ ID NO.255-301) were synthesized by Sangon Biotech (Shanghai) Co., Ltd.

According to the same procedures of Embodiment 15, after annealing the corresponding primers, the 47 target sequence DNAs were inserted into the pAcC2C9Hs plasmid using Golden Gate assembly technology. Subsequently, the successfully constructed plasmid was transfected into HEK293 cells for gene editing using lipofectamine3000 (Invitrogen). The edited cells were collected to extract the genome, and the products were sent to Jiangxi HaploX Biotechnology Co. Ltd. for high-throughput sequencing after amplification of the edited sites to test the efficiency of cell editing.

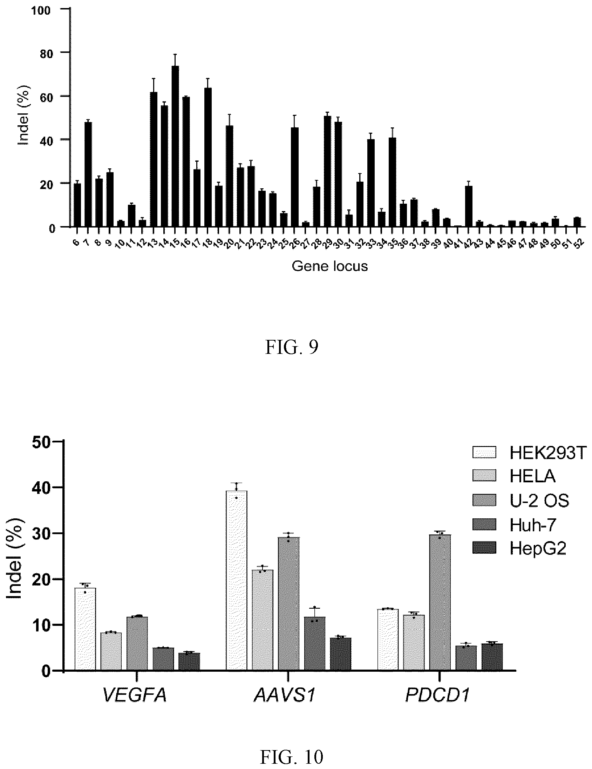

Figure 9 shows the genome editing efficiency of the transient expression plasmid of AcC2C9 at 47 loci in HEK293T cells. The highest gene editing efficiency was at site 15, with an efficiency of 73.7%.

### Embodiment 17 Gene editing of adeno-associated virus (AAV)-mediated AcC2C9 in human live cells

The full gene synthesis of the AAV2 plasmid backbone (the sequence of which is shown as SEQ ID NO.302) and the AcC2C9 expression cassettes targeting the VEGFA, AAVS1, and PDCD1 genes (the sequences of which are shown as SEQ ID NO.303-305) was performed by Sangon Biotech (Shanghai) Co., Ltd. The AAV2 plasmid backbone was assembled with three AcC2C9 expression cassettes using Gibson assembly technology to obtain AAV2-AcVEGFA(the sequence is shown as SEQ ID NO. 306), AAV2-AcAAVS1 (the sequence is shown as SEQ ID NO. 307) and AAV2-AcPDCD1(the sequence is shown as SEQ ID NO. 308). The plasmids were transformed into competent Escherichia coli DH5αcells, coated on the LB medium plate containing 50µg/mL carbenicillin for selection. The single colonies were picked for expansion culture, the plasmids were extracted and sequenced to identify plasmid sequences.

The constructed plasmids were sent to Guangzhou PackGene Biotech Co. Ltd. for AAV production, and AAVs containing the AcC2C9 system targeting three different loci were obtained. Cryopreserved HEK293T, HLEA, U-2 OS, Huh-7, and HepG cells were thawed and activated. The human cells were passaged into a 24-well plate with approximately 0.5 × 10⁵ cells per well. After 24 hours, 0.5 × 10¹⁰ copies of AAV were added to each well for transfection. After culturing for 72 hours, the adherent cells were digested and genomic DNA was extracted. After amplifying the edited sites, the products were sent to Jiangxi HaploX Biotechnology Co. for high-throughput sequencing to detect cell editing efficiency.

Figure 10 shows the editing efficiency of AAV expressing the AcC2C9 system at three different gene loci (VEGFA, AAVS1, PDCD1) in human cells HEK293T, HLEA, U-2 OS, Huh-7, and HepG. The editing efficiencies of AAV expressing the AcC2C9 system were 18.2%, 39.4%, and 13.5% at three different gene loci in human cells HEK293T cells, and the editing efficiencies of AAV expressing the AcC2C9 system were 8.4%, 22.1%, and 12.2% at three different gene loci in human cells HLEA cells. The editing efficiencies of AAV expressing the AcC2C9 system were 11.8%, 29.2%, and 29.8% at three different gene loci in human cells U-2 OS cells. The editing efficiencies of AAV expressing the AcC2C9 system were 5.1%, 11.9%, and 5.6% at three different loci in human Huh-7 cells. The editing efficiencies of AAV expressing the AcC2C9 system were 3.9%, 7.4%, and 6.0% at three different gene loci in human HepG cells.

The above embodiments are intended only to illustrate the technical solution of the present disclosure and not to limit it. Although the present disclosure is described in detail with reference to the preferred embodiment, the person of ordinary skill in the field should understand that it is still possible to modify the specific embodiment of the present disclosure or make equivalent substitutions for some of the technical features. Without departing from the spirit and scope of the inventive concept, the variations and advantages that can be thought of by the person skilled in the art shall be covered in the scope of the technical solutions for which protection is sought by the present disclosure.

## Claims

1. A use of a C2C9 nuclease as an RNA-guided endonuclease.

2. A use of a C2C9 nuclease or a nucleic acid encoding the C2C9 nuclease in a gene editing system.

3. A gene editing system, comprising a C2C9 nuclease and/or a nucleic acid encoding the C2C9 nuclease, and a guide RNA or a nucleic acid encoding the guide RNA.

4. The gene editing system of claim 3, wherein the C2C9 nuclease is:
(I) a wild-type C2C9 nuclease or fragments thereof, with a RNA-guided nucleic acid binding activity;
(II) a variant having at least 30% sequence identity to an amino acid sequence of (I) and having a RNA-guided nucleic acid binding activity;
(III) according to (I) or (II), a nuclear localization signal fragment is included;
(IV) according to (I), (II), or (III), one or more of the following is included:
(a) one or more modifications or mutations, resulting in a significantly reduced endonuclease activity, or a loss of endonuclease activity; and
(b) a polypeptide or domain with other functional activities;
(V) according to (I), (II), or (III), the C2C9 nuclease has an endonuclease activity.

5. The gene editing system of claim 3 or 4, wherein the C2C9 nuclease has no more than 800 amino acids; and/or, the C2C9 nuclease has the RNA-guided nucleic acid binding activity.

6. The gene editing system of any one of claims 3-5, wherein the gene editing system recognizes a PAM sequence on a target sequence; and/or, the gene editing system targets a nucleic acid fragment of 12 to 40 bp in length following the PAM sequence, preferably, the gene editing system targets a nucleic acid fragment of 20 bp in length following the PAM sequence.

7. The gene editing system of claim 6, wherein the PAM sequence is AAN or GAN; wherein N is a degenerate base and selected from A, T, C, and G.

8. The gene editing system of any one of claims 3-7, wherein the C2C9 nuclease, from the N-terminus to the C-terminus, comprises:
a domain 1, which comprises an amino acid sequence shown as SEQ ID NO. 1 or a variant having at least 30% sequence identity to SEQ ID NO. 1, and has a RNA-guided nucleic acid binding activity and/or endonuclease activity;
a domain 2, which comprises an amino acid sequence shown as SEQ ID NO. 2 or a variant having at least 30% sequence identity to SEQ ID NO. 2, and has a RNA-guided nucleic acid binding activity and/or endonuclease activity.

9. The gene editing system of any one of claims 3-7, wherein the C2C9 nuclease has any one of the amino acid sequences shown as SEQ ID NOs. 3-117 or has at least 30% sequence identity to any one of the amino acid sequences shown as SEQ ID NOs. 3-117, and has a RNA-guided nucleic acid binding activity and/or endonuclease activity.

10. The gene editing system of any one of claims 3-7, wherein the guide RNA comprises:
a gene-targeting segment (i), that is capable of hybridizing to the target sequence;
a tracr pairing sequence (ii); and
a tracr RNA sequence (iii);
wherein the tracr pairing sequence (ii) hybridizes to the tracr RNA sequence (iii) and forms a stem-loop structure;
wherein the guide RNA is a single strand which is formed by sequentially linking of the gene targeting segment (i) to the tracr pairing sequence (ii) and the tracr RNA sequence (iii); or,
the guide RNA comprises two strands, wherein one strand is formed by linking the gene-targeting segment (i) to the tracr pairing sequence (ii) and the other strand is the tracr RNA sequence (iii).

11. The gene editing system of claim 5 or 6, wherein the gene editing system comprises:
(1) an expression construct for the C2C9 nuclease;
(2) an expression construct for a complete guide RNA;
wherein the complete guide RNA comprises a guide RNA backbone, and a RNA sequence corresponding to the target sequence added at the 3' end of the guide RNA backbone;
wherein the target sequence is a nucleic acid fragment having 12 to 40 bp in length following the PAM sequence, preferably the target sequence is a nucleic acid fragment having 20 bp in length following the PAM sequence.

12. The gene editing system of claim 10, wherein the guide RNA corresponds to the C2C9 nuclease, wherein the tracr pairing sequence corresponding to *Actinomadura craniellae* C2C9 (SEQ ID NO. 3) is shown as SEQ ID NO. 118, and the tracr RNA sequence is shown as SEQ ID NO. 119, and a sequence of the guide RNA backbone obtained by linking the tracr RNA sequence to the tracr pairing sequence is shown as SEQ ID NO. 120.

13. A gene editing method, comprising making a target gene in contact with the gene editing system of any one of claims 3-12 to perform editing of the target gene.

14. The gene editing method of claim 13, comprising the following steps:
i) introducing the C2C9 nuclease or the nucleic acid encoding the C2C9 nuclease into a cell.
ii) introducing the guide RNA or the nucleic acid encoding the guide RNA into the cell;
iii) producing one or more nicks in the target gene, or targeting, editing, modifying, or manipulating the target gene by the C2C9 nuclease.

15. The gene editing method of claim 13 or 14, wherein the C2C9 nuclease is directed to the target gene by the guide RNA in a processed or unprocessed form.

16. The gene editing method of claim 13 or 14, wherein the C2C9 nuclease and the guide RNA form a complex that recognizes the PAM sequence on the target gene; and/or, the target sequence of the gene editing system is a nucleic acid fragment with 12 to 40 bp in length following the PAM sequence, preferably target sequence is a nucleic acid fragment with 20 bp in length following the PAM sequence.

17. The gene editing method of claim 14, further comprising a step of introducing a donor template comprising a heterologous polynucleotide sequence into the cell.

18. A use of the gene editing system of any one of claims 3-12 or the method of any one of claims 13-17 for gene editing of a target gene and/or its associated polypeptide in vivo, in an isolated cell, or in a cell-free environment, wherein the isolated cell comprises bacterial cells, archaeal cells, fungal cells, protozoan cells, viral cells, plant cells, and animal cells.

19. A use of the gene editing system of any one of claims 3-12 or the method of any one of claims 13-17 in gene editing of a target gene and/or its associated polypeptide in vivo, in an isolated cell, or in a cell-free environment, wherein the gene editing is selected from a group consisting of: gene cleavage, gene deletion, gene insertion, point mutation, transcription inhibition, transcription activation, base editing, and prime editing.

20. A genetically modified cell, which is obtained by gene editing of the gene editing system according to any one of claims 3-12 or the method according to any one of claims 13-17.
